(19) Europäisches Patentamt
European Patent Office
Office européen des brevets

(11) Veröffentlichungsnummer : **0 608 198 A1**

(12) # EUROPÄISCHE PATENTANMELDUNG

(21) Anmeldenummer : **94810012.8**

(51) Int. Cl.[5] : **C08K 5/00,** C07C 255/47

(22) Anmeldetag : **11.01.94**

(30) Priorität : **18.01.93 CH 129/93**

(43) Veröffentlichungstag der Anmeldung :
**27.07.94 Patentblatt 94/30**

(84) Benannte Vertragsstaaten :
**BE DE ES FR GB IT NL**

(71) Anmelder : **CIBA-GEIGY AG**
**Klybeckstrasse 141**
**CH-4002 Basel (CH)**

(72) Erfinder : **Pitteloud, Rita Dr.**
**Sur le Village**
**CH-1724 Praroman (CH)**

(54) **Cyclische Diphenylacetonitrile als Stabilisatoren.**

(57) Es werden Verbindungen der Formel I,

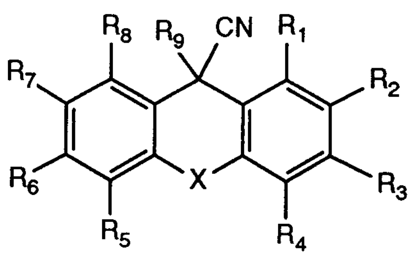

(I)

worin die allgemeinen Symbole wie in Anspruch 1 definiert sind, als Stabilisatoren für organische Materialien gegen thermischen, oxidativen oder lichtinduzierten Abbau beschrieben.

EP 0 608 198 A1

Jouve, 18, rue Saint-Denis, 75001 PARIS

Die vorliegende Erfindung betrifft Zusammensetzungen, enthaltend ein organisches Material, bevorzugt ein Polymer, und cyclische Diphenylacetonitrile als Stabilisatoren, die Verwendung derselben zum Stabilisieren von organischen Materialien gegen oxidativen, thermischen oder lichtinduzierten Abbau sowie neue cyclische Diphenylacetonitrile.

Einzelne cyclische Diphenylacetonitile sind in der Literatur bekannt, und wurden beispielsweise von M. N. Romanelli et al, 11 Farmaco 46 (10), 1121 (1991); M. Hori et al, J. Org. Chem. 45 (12), 2468 (1980); V. Valenta et al, Coll. Czech. Chem. Comm. 53 (4), 860 (1988); M.A. Davis et al, J. Med. Chem. 7, 88 (1964); A. Kaufmann et al, Ber. Dt. Chem. Ges. 42, 1999 (1909); und in US-A-3 452 076 oder US-A-2 956 063 beschrieben. In keiner Publikation werden diese Verbindungen als Stabilisatoren für organische Materialien verwendet.

Die Verwendung von einigen Dibenzopyranen als Stabilisatoren für organische Materialien wurde beispielsweise in JP-A-60 084 383 und JP-A-60 088 087 beschrieben.

Es wurde nun gefunden, dass eine ausgewählte Gruppe cyclischer Diphenylacetonitrile sich besonders gut als Stabilisatoren für organische Materialien, die gegen oxidativen, thermischen oder lichtinduzierten Abbau empfindlich sind, eignen.

Die vorliegende Erfindung betrifft daher Zusammensetzungen enthaltend

a) ein dem oxidativen, thermischen oder lichtinduzierten Abbau unterworfenes organisches Material und

b) mindestens eine Verbindung der Formel I,

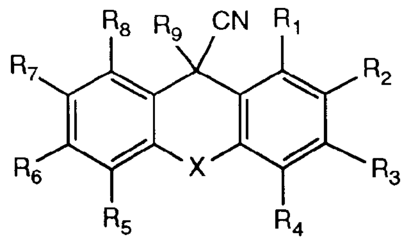

(I)

worin $R_1$, $R_2$, $R_3$, $R_4$, $R_5$, $R_6$, $R_7$ und $R_8$ unabhängig voneinander Wasserstoff, Halogen, $C_1$-$C_{25}$-Alkyl, durch Sauerstoff, Schwefel oder $>N-R_{10}$ unterbrochenes $C_2$-$C_{25}$-Alkyl; unsubstituiertes oder durch $C_1$-$C_4$-Alkyl substituiertes $C_5$-$C_8$-Cycloalkyl; unsubstituiertes oder durch $C_1$-$C_4$-Alkyl substituiertes Phenyl; $C_7$-$C_9$-Phenylalkyl, Hydroxy, $C_1$-$C_{18}$-Alkoxy, durch Sauerstoff, Schwefel oder $>N-R_{10}$ unterbrochenes $C_3$-$C_{18}$-Alkoxy; Mercapto, $C_1$-$C_{18}$-Alkylthio, durch Sauerstoff, Schwefel oder $>N-R_{10}$ unterbrochenes $C_3$-$C_{18}$-Alkylthio; $C_1$-$C_{25}$-Alkanoyloxy, durch Sauerstoff, Schwefel oder $>N-R_{10}$ unterbrochenes $C_3$-$C_{25}$-Alkanoyloxy; $C_3$-$C_{25}$-Alkenoyloxy, Benzoyloxy oder durch $C_1$-$C_{12}$-Alkyl substituiertes Benzoyloxy; Nitro, Cyano, $-(CH_2)_mCOR_{11}$ oder

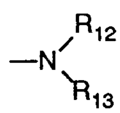

bedeuten, ferner die Reste $R_1$ und $R_2$, $R_2$ und $R_3$, $R_3$ und $R_4$, $R_5$ und $R_6$, $R_5$ und $R_7$ oder $R_7$ und $R_8$ zusammen mit den Kohlenstoffatomen, an die sie gebunden sind, einen Benzoring bilden, oder die gleichen Rest-Paare zusammen $-O(CH_2)_nO-$ bedeuten, mit der Bedingung, dass mindestens einer der Reste $R_1$, $R_2$, $R_3$ oder $R_4$, sowie mindestens einer der Reste $R_5$, $R_6$, $R_7$ oder $R_8$ Wasserstoff darstellen, $R_1$ zusätzlich einen Rest der Formel II, $R_2$ zusätzlich einen Rest der Formel III, $R_3$ zusätzlich einen Rest der Formel IV und $R_4$ zusätzlich einen Rest der Formel V bedeutet, wobei gleichzeitig nur ein Rest der Formel II, III, IV oder V in der Verbindung der Formel I auftritt,

(II)

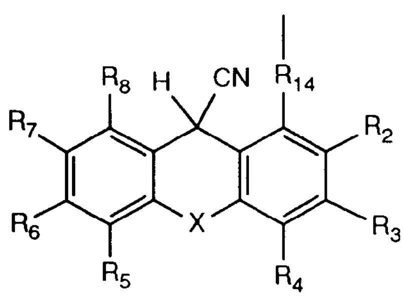

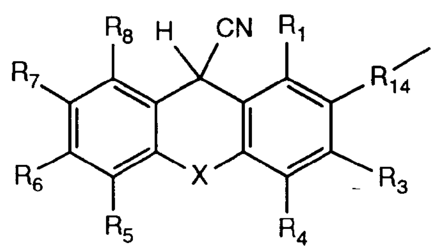

(III)

(IV) 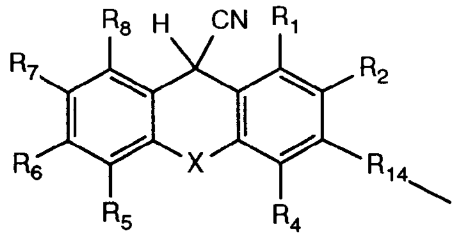 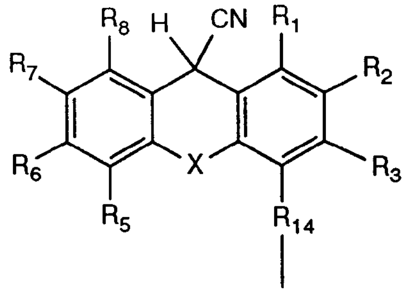 (V)

$R_9$ Wasserstoff oder einen Rest der Formel VI

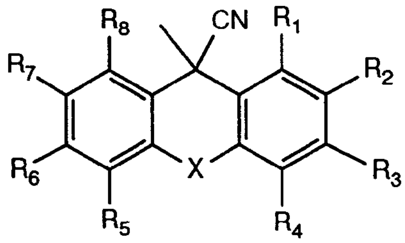 (VI)

darstellt, wobei $R_1$, $R_2$, $R_3$ und $R_4$ nicht einen Rest der Formel II, III, IV oder V bedeuten,
$R_{10}$ Wasserstoff oder $C_1$-$C_8$-Alkyl darstellt,
$R_{11}$ Hydroxy, $C_1$-$C_{18}$-Alkoxy oder

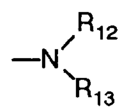

bedeutet,
$R_{12}$ und $R_{13}$ unabhängig voneinander Wasserstoff oder $C_1$-$C_{18}$-Alkyl darstellen,
$R_{14}$ eine direkte Bindung, $C_1$-$C_{18}$-Alkylen, durch Sauerstoff, Schwefel oder >N-$R_{10}$ unterbrochenes $C_2$-$C_{18}$-Alkylen;

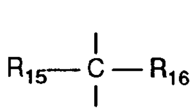 oder 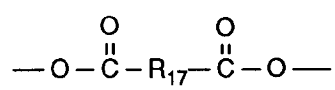

bedeutet,
$R_{15}$ und $R_{16}$ unabhängig voneinander Wasserstoff, $CF_3$, $C_1$-$C_{12}$-Alkyl oder Phenyl darstellen, oder $R_{15}$ und $R_{16}$ zusammen mit dem C-Atom, an das sie gebunden sind, einen unsubstituierten oder durch 1 bis 3 $C_1$-$C_4$-Alkyl substituierten $C_5$-$C_8$-Cycloalkylidenring bilden,
$R_{17}$ eine direkte Bindung, $C_1$-$C_{18}$-Alkylen, durch Sauerstoff, Schwefel oder >N-$R_{10}$ unterbrochenes $C_2$-$C_{18}$-Alkylen; $C_2$-$C_{18}$-Alkenylen, $C_2$-$C_{20}$-Alkyliden, $C_7$-$C_{20}$-Phenylalkyliden, $C_5$-$C_8$-Cycloalkylen, $C_7$-$C_8$-Bicycloalkylen, unsubstituiertes oder durch $C_1$-$C_4$-Alkyl substituiertes Phenylen,

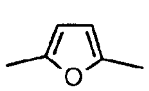 oder 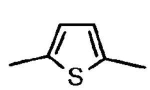

darstellt,
$R_{18}$ Wasserstoff, $C_1$-$C_{25}$-Alkyl, durch Sauerstoff, Schwefel oder >N-$R_{10}$ unterbrochenes $C_2$-$C_{25}$-Alkyl; unsubstituiertes oder durch $C_1$-$C_4$-Alkyl substituiertes $C_5$-$C_8$-Cycloalkyl; unsubstituiertes oder durch $C_1$-$C_4$-Alkyl substituiertes Phenyl; $C_7$-$C_9$-Phenylalkyl, Hydroxy, $C_1$-$C_{18}$-Alkoxy, durch Sauerstoff, Schwefel oder >N-$R_{10}$ un-

terbrochenes $C_3$-$C_{18}$-Alkoxy; $C_1$-$C_{25}$-Alkanoyl, durch Sauerstoff, Schwefel oder >N-$R_{10}$ unterbrochenes $C_3$-$C_{25}$-Alkanoyl; $C_3$-$C_{25}$-Alkenoyl, Benzoyl oder durch $C_1$-$C_{12}$-Alkyl substituiertes Benzoyl; Cyano, $-(CH_2)_mCOR_{11}$ oder

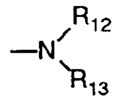

bedeutet,
$R_{19}$ $C_1$-$C_{25}$-Alkyl, $C_7$-$C_9$-Phenylalkyl oder $-CH_2CH_2OH$ darstellt,
X eine direkte Bindung, Sauerstoff, Schwefel, -SO-, $-SO_2-$, $-CH_2-$, $-CH_2CH_2-$, -CH=CH-,

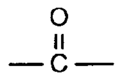 , 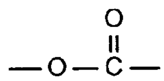 , 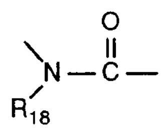 , 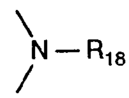 , 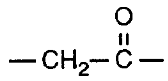

oder

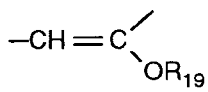

bedeutet,
m 0, 1 oder 2, und
n 1 oder 2 darstellt.

Halogen bedeutet beispielsweise Chlor, Brom oder Iod. Bevorzugt ist Chlor.

Alkyl mit bis zu 25 Kohlenstoffatomen bedeutet einen verzweigten oder unverzweigten Rest wie beispielsweise Methyl, Ethyl, Propyl, Isopropyl, n-Butyl, sec-Butyl, Isobutyl, tert-Butyl, 2-Ethylbutyl, n-Pentyl, Isopentyl, 1-Methylpentyl, 1,3-Dimethylbutyl, n-Hexyl, 1-Methylhexyl, n-Heptyl, Isoheptyl, 1,1,3,3-Tetramethylbutyl, 1-Methylheptyl, 3-Methylheptyl, n-Octyl, 2-Ethylhexyl, 1,1,3-Trimethylhexyl, 1,1,3,3-Tetramethylpentyl, Nonyl, Decyl, Undecyl, 1-Methylundecyl, Dodecyl, 1,1,3,3,5,5-Hexamethylhexyl, Tridecyl, Tetradecyl, Pentadecyl, Hexadecyl, Heptadecyl, Octadecyl, Eicosyl oder Docosyl. Eine bevorzugte Bedeutung von $R_2$ ist $C_1$-$C_{18}$-Alkyl, insbesondere $C_1$-$C_{12}$-Alkyl. Eine besonders bevorzugte Bedeutung von $R_4$ ist $C_1$-$C_8$-Alkyl, insbesondere $C_1$-$C_4$-Alkyl.

Durch Sauerstoff, Schwefel oder >N-$R_{10}$ unterbrochenes $C_2$-$C_{25}$-Alkyl bedeutet beispielsweise $CH_3$-O-$CH_2$-, $CH_3$-S-$CH_2$-, $CH_3$-NH-$CH_2$-, $CH_3$-N($CH_3$)-$CH_2$-, $CH_3$-O-$CH_2CH_2$-O-$CH_2$-, $CH_3$-(O-$CH_2CH_2$-$)_2$O-$CH_2$-, $CH_3$-(O-$CH_2CH_2$-$)_3$O-$CH_2$- oder $CH_3$-(O-$CH_2CH_2$-$)_4$O-$CH_2$-.

Unsubstituiertes oder durch $C_1$-$C_4$-Alkyl substituiertes $C_5$-$C_8$-Cycloalkyl, insbesondere $C_5$-$C_6$-Cycloalkyl, bedeutet beispielsweise Cyclopentyl, Methylcyclopentyl, Dimethylcyclopentyl, Cyclohexyl, Methylcyclohexyl, Dimethylcyclohexyl, Trimethylcyclohexyl, tert-Butylcyclohexyl, Cycloheptyl oder Cyclooctyl. Bevorzugt ist Cyclohexyl und tert-Butylcyclohexyl.

Durch $C_1$-$C_4$-Alkyl substituiertes Phenyl, das vorzugsweise 1 bis 3, insbesondere 1 oder 2 Alkylgruppen enthält, bedeutet beispielsweise o-, m- oder p-Methylphenyl, 2,3-Dimethylphenyl, 2,4-Dimethylphenyl, 2,5-Dimethylphenyl, 2,6-Dimethylphenyl, 3,4-Dimethylphenyl, 3,5-Dimethylphenyl, 2-Methyl-6-ethylphenyl, 4-tert-butylphenyl, 2-Ethylphenyl oder 2,6-Diethylphenyl.

$C_7$-$C_9$-Phenylalkyl bedeutet beispielsweise Benzyl, α-Methylbenzyl, α,α-Dimethylbenzyl oder 2-Phenylethyl.

Alkoxy mit bis zu 18 Kohlenstoffatomen bedeutet einen verzweigten oder unverzweigten Rest wie beispielsweise Methoxy, Ethoxy, Propoxy, Isopropoxy, n-Butoxy, Isobutoxy, Pentoxy, Isopentoxy, Hexoxy, Heptoxy, Octoxy, Decyloxy, Tetradecyloxy, Hexadecyloxy oder Octadecyloxy. Eine bevorzugte Bedeutung von $R_2$ ist $C_1$-$C_6$-Alkoxy, insbesondere $C_1$-$C_3$-Alkoxy. Eine bevorzugte Bedeutung von $R_{11}$ ist $C_1$-$C_{12}$-Alkoxy.

Durch Sauerstoff, Schwefel oder >N-$R_{10}$ unterbrochenes $C_3$-$C_{18}$-Alkoxy bedeutet beispielsweise $CH_3$-O-$CH_2CH_2$O-, $CH_3$-S-$CH_2CH_2$O-, $CH_3$-NH-$CH_2CH_2$O-, $CH_3$-N($CH_3$)-$CH_2CH_2$O-, $CH_3$-O-$CH_2CH_2$-O-$CH_2CH_2$O-, $CH_3$-(O-$CH_2CH_2$-$)_2$O-$CH_2CH_2$O, $CH_3$-(O-$CH_2CH_2$-$)_3$O-$CH_2CH_2$O- oder $CH_3$-(O-$CH_2CH_2$-$)_4$O-$CH_2CH_2$O-.

Alkylthio mit bis zu 18 Kohlenstoffatomen bedeutet einen verzweigten oder unverzweigten Rest wie beispielsweise Methylthio, Ethylthio, Propylthio, Isopropylthio, n-Butylthio, Isobutylthio, Pentylthio, Isopentylthio,

Hexylthio, Heptylthio, Octylthio, Decylthio, Tetradecylthio, Hexadecylthio oder Octadecylthio. Bevorzugt ist Alkylthio mit 1 bis 12, insbesondere 1 bis 8, z.B. 1 bis 6 Kohlenstoffatomen.

Durch Sauerstoff, Schwefel oder >N-$R_{10}$ unterbrochenes $C_3$-$C_{18}$-Alkylthio bedeutet beispielsweise $CH_3$-O-$CH_2CH_2S$-, $CH_3$-S-$CH_2CH_2S$-, $CH_3$-NH-$CH_2CH_2S$-, $CH_3$-N($CH_3$)-$CH_2CH_2S$-, $CH_3$-O-$CH_2CH_2$-O-$CH_2CH_2S$-, $CH_3$-(O-$CH_2CH_2$-$)_2$O-$CH_2CH_2S$-, $CH_3$-(O-$CH_2CH_2$-$)_3$O-$CH_2CH_2S$- oder $CH_3$-(O-$CH_2CH_2$-$)_4$O-$CH_2CH_2S$-.

Alkanoyloxy mit bis zu 25 Kohlenstoffatomen bedeutet einen verzweigten oder unverzweigten Rest wie beispielsweise Formyloxy, Acetyloxy, Propionyloxy, Butanoyloxy, Pentanoyloxy, Hexanoyloxy, Heptanoyloxy, Octanoyloxy, Nonanoyloxy, Decanoyloxy, Undecanoyloxy, Dodecanoyloxy, Tridecanoyloxy, Tetradecanoylxoy, Pentadecanoyloxy, Hexadecanoyloxy, Heptadecanoyloxy, Octadecanoyloxy, Eicosanoyloxy oder Docosanoyloxy. Bevorzugt ist Alkanoyloxy mit 2 bis 18, insbesondere 2 bis 12 Kohlenstoffatomen.

Durch Sauerstoff, Schwefel oder >N-$R_{10}$ unterbrochenes $C_3$-$C_{25}$-Alkanoyloxy bedeutet beispielsweise $CH_3$-O-$CH_2$COO-, $CH_3$-S-$CH_2$COO-, $CH_3$-NH-$CH_2$COO-, $CH_3$-N($CH_3$)-$CH_2$COO-, $CH_3$-O-$CH_2CH_2$-O-$CH_2$COO-, $CH_3$-(O-$CH_2CH_2$-$)_2$0OCH$_2$COO-, $CH_3$-(O-$CH_2CH_2$-$)_3$O-$CH_2$COO- oder $CH_3$-(O-$CH_2CH_2$-$)_4$O-$CH_2$COO-.

Alkenoyloxy mit 3 bis 25 Kohlenstoffatomen bedeutet einen verzweigten oder unverzweigten Rest wie beispielsweise Propenoyloxy, 2-Butenoyloxy, 3-Butenoyloxy, Isobutenoyloxy, n-2,4-Pentadienoyloxy, 3-Methyl-2-butenoyloxy, n-2-Octenoyloxy, n-2-Dodecenoyloxy, iso-Dodecenoyloxy, Oleoyloxy, n-2-Octadecenoyloxy oder n-4-Octadecenoyloxy. Bevorzugt ist Alkenoyloxy mit 3 bis 18, insbesondere 3 bis 12, z.B. 3 bis 6, vor allem 3 bis 4 Kohlenstoffatomen.

Durch $C_1$-$C_{12}$-Alkyl substituiertes Benzoyloxy, das vorzugsweise 1 bis 3, insbesondere 1 oder 2 Alkylgruppen trägt, bedeutet beispielsweise o-, m- oder p-Methylbenzoyloxy, 2,3-Dimethylbenzoyloxy, 2,4-Dimethylbenzoyloxy, 2,5-Dimethylbenzoyloxy, 2,6-Dimethylbenzoyloxy, 3,4-Dimethylbenzoyloxy, 3,5-Dimethylbenzoyloxy, 2-Methyl-6-ethylbenzoyloxy, 4-tert-Butylbenzoyloxy, 2-Ethylbenzoyloxy, 2,4,6-Trimethylbenzoyloxy, 2,6-Dimethyl-4-tert-butylbenzoyloxy oder 3,5-Di-tert-butylbenzoyloxy. Bevorzugte Substituenten sind $C_1$-$C_8$-Alkyl, insbesondere $C_1$-$C_4$-Alkyl.

$C_1$-$C_{18}$-Alkylen bedeutet einen verzweigten oder unverzweigten Rest wie beispielsweise Methylen, Ethylen, Propylen, Trimethylen, Tetramethylen, Pentamethylen, Hexamethylen, Heptamethylen, Octamethylen, Decamethylen, Dodecamethylen oder Octadecamethylen. Bevorzugt ist $C_1$-$C_{12}$-Alkylen, insbesondere $C_1$-$C_8$-Alkylen.

Durch Sauerstoff, Schwefel oder >N-$R_{10}$ unterbrochenes $C_2$-$C_{18}$-Alkylen bedeutet beispielsweise -$CH_2$-O-$CH_2$-, -$CH_2$-S-$CH_2$-, -$CH_2$-NH-$CH_2$-, -$CH_2$-N($CH_3$)-$CH_2$-, -$CH_2$-O-$CH_2CH_2$-O-$CH_2$-, -$CH_2$-(O-$CH_2CH_2$-$)_2$O-$CH_2$-, -$CH_2$-(O-$CH_2CH_2$-$)_3$O-$CH_2$- , -$CH_2$-(O-$CH_2CH_2$-$)_4$O-$CH_2$- oder -$CH_2CH_2$-S-$CH_2CH_2$-.

Ein unsubstituierter oder durch $C_1$-$C_4$-Alkyl substituierter $C_5$-$C_8$-Cycloalkylidenring, der vorzugsweise 1 bis 3, insbesondere 1 oder 2 verzweigte oder unverzweigte Alkylgruppen-Reste enthält, bedeutet beispielsweise Cyclopentyliden, Methylcyclopentyliden, Dimethylcyclopentyliden, Cyclohexyliden, Methylcyclohexyliden, Dimethylcyclohexyliden, Trimethylcyclohexyliden, tert-Butylcyclohexyliden, Cycloheptyliden oder Cyclooctyliden. Bevorzugt ist Cyclohexyliden und tert-Butylcyclohexyliden.

$C_2$-$C_{18}$-Alkenylen bedeutet beispielsweise Vinylen, Methylvinylen, Octenylethylen oder Dodecenylethylen. Bevorzugt ist $C_2$-$C_8$-Alkenylen.

Alkyliden mit 2 bis 20 Kohlenstoffatomen bedeutet beispielsweise Ethyliden, Propyliden, Butyliden, Pentyliden, 4-Methylpentyliden, Heptyliden, Nonyliden, Tridecyliden, Nonadecyliden, 1-Methylethyliden, 1-Ethylpropyliden oder 1-Ethylpentyliden. Bevorzugt ist $C_2$-$C_8$-Alkyliden.

Phenylalkyliden mit 7 bis 20 Kohlenstoffatomen bedeutet beispielsweise Benzyliden, 2-Phenylethyliden oder 1-Phenyl-2-hexyliden. Bevorzugt ist $C_7$-$C_9$-Phenylalkyliden.

$C_5$-$C_8$-Cycloalkylen bedeutet eine gesättigte Kohlenwasserstoffgruppe mit zwei freien Valenzen und mindestens einer Ringeinheit und ist beispielsweise Cyclopentylen, Cyclohexylen, Cycloheptylen oder Cyclooctylen. Bevorzugt ist Cyclohexylen.

$C_7$-$C_8$-Bicycloalkylen bedeutet beispielsweise Bicycloheptylen oder Bicyclooctylen.

Unsubstituiertes oder durch $C_1$-$C_4$-Alkyl substituiertes Phenylen bedeutet beispielsweise 1,2-, 1,3- oder 1,4-Phenylen. 1,4-Phenylen ist bevorzugt.

Alkanoyl mit bis zu 25 Kohlenstoffatomen bedeutet einen verzweigten oder unverzweigten Rest wie beispielsweise Formyl, Acetyl, Propionyl, Butanoyl, Pentanoyl, Hexanoyl, Heptanoyl, Octanoyl, Nonanoyl, Decanoyl, Undecanoyl, Dodecanoyl, Tridecanoyl, Tetradecanoyl, Pentadecanoyl, Hexadecanoyl, Heptadecanoyl, Octadecanoyl, Eicosanoyl oder Docosanoyl. Bevorzugt ist Alkanoyl mit 2 bis 18, insbesondere 2 bis 12, z.B. 2 bis 6 Kohlenstoffatomen. Besonders bevorzugt ist Acetyl.

Durch Sauerstoff, Schwefel oder >N-$R_{10}$ unterbrochenes $C_3$-$C_{25}$-Alkanoyl bedeutet beispielsweise $CH_3$-O-$CH_2$CO-, $CH_3$-S-$CH_2$CO-, $CH_3$-NH-$CH_2$CO-, $CH_3$-N($CH_3$)-$CH_2$CO-, $CH_3$-O-$CH_2CH_2$-O-$CH_2$CO-, $CH_3$-(O-

$CH_2CH_2$-$)_2O$-$CH_2CO$-, $CH_3$-(O-$CH_2CH_2$-$)_3O$-$CH_2CO$- oder $CH_3$-(O-$CH_2CH_2$-$)_4O$-$CH_2CO$-.

Alkenoyl mit 3 bis 25 Kohlenstoffatomen bedeutet einen verzweigten oder unverzweigten Rest wie beispielsweise Propenoyl, 2-Butenoyl, 3-Butenoyl, Isobutenoyl, n-2,4-Pentadienoyl, 3-Methyl-2-butenoyl, n-2-Octenoyl, n-2-Dodecenoyl, iso-Dodecenoyl, Oleoyl, n-2-Octadecenoyl oder n-4-Octadecenoyl. Bevorzugt ist Alkenoyl mit 3 bis 18, insbesondere 3 bis 12, z.B. 3 bis 6, vor allem 3 bis 4 Kohlenstoffatomen.

Durch $C_1$-$C_{12}$-Alkyl substituiertes Benzoyl, das vorzugsweise 1 bis 3, insbesondere 1 oder 2 Alkylgruppen trägt, bedeutet beispielsweise o-, m- oder p-Methylbenzoyl, 2,3-Dimethylbenzoyl, 2,4-Dimethylbenzoyl, 2,5-Dimethylbenzoyl, 2,6-Dimethylbenzoyl, 3,4-Dimethylbenzoyl, 3,5-Dimethylbenzoyl, 2-Methyl-6-ethylbenzoyl, 4-tert-Butylbenzoyl, 2-Etyhlbenzoyl, 2,4,6-Trimethylbenzoyl, 2,6-Dimethyl-4-tert-butylbenzoyl oder 3,5-Di-tert-butylbenzoyl. Bevorzugte Substituenten sind $C_1$-$C_8$-Alkyl, insbesondere $C_1$-$C_4$-Alkyl.

Von Interesse sind Zusammensetzungen enthaltend Verbindungen der Formel I, worin $R_1$, $R_2$, $R_3$, $R_4$, $R_5$, $R_6$, $R_7$ und $R_8$ unabhängig voneinander Wasserstoff, Chlor, $C_1$-$C_{18}$-Alkyl, durch Sauerstoff, Schwefel oder >N-$R_{10}$ unterbrochenes $C_2$-$C_{18}$-Alkyl; unsubstituiertes oder durch $C_1$-$C_4$-Alkyl substituiertes $C_5$-$C_8$-Cycloalkyl; unsubstituiertes oder durch $C_1$-$C_4$-Alkyl substituiertes Phenyl; $C_7$-$C_9$-Phenylalkyl, Hydroxy, $C_1$-$C_{18}$-Alkoxy, durch Sauerstoff, Schwefel oder >N-$R_{10}$ unterbrochenes $C_3$-$C_{18}$-Alkoxy; Mercapto, $C_1$-$C_{18}$-Alkylthio, $C_1$-$C_{18}$-Alkanoyloxy, durch Sauerstoff, Schwefel oder >N-$R_{10}$ unterbrochenes $C_3$-$C_{18}$-Alkanoyloxy; $C_3$-$C_{18}$-Alkenoyloxy, Benzoyloxy oder durch $C_1$-$C_8$-Alkyl substituiertes Benzoyloxy; Nitro, Cyano, -$(CH_2)_mCOR_{11}$ oder

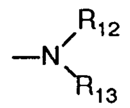

bedeuten, ferner die Reste $R_1$ und $R_2$, $R_2$ und $R_3$, $R_3$ und $R_4$, $R_5$ und $R_6$, $R_5$ und $R_7$ oder $R_7$ und $R_8$ zusammen mit den Kohlenstoffatomen, an die sie gebunden sind, einen Benzoring bilden, oder die gleichen Rest-Paare zusammen -$O(CH_2)_nO$- bedeuten, mit der Bedingung, dass mindestens einer der Reste $R_1$, $R_2$, $R_3$ oder $R_4$, sowie mindestens einer der Reste $R_5$, $R_6$, $R_7$ oder $R_8$ Wasserstoff darstellen, $R_1$ zusätzlich einen Rest der Formel II, $R_2$ zusätzlich einen Rest der Formel III, $R_3$ zusätzlich einen Rest der Formel IV und $R_4$ zusätzlich einen Rest der Formel V bedeutet, wobei gleichzeitig nur ein Rest der Formel II, III, IV oder V in der Verbindung der Formel I auftritt,

(II) 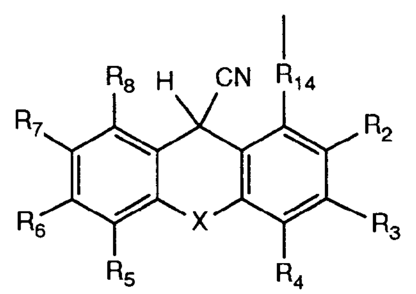

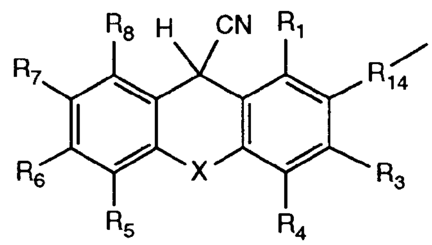 (III)

(IV) 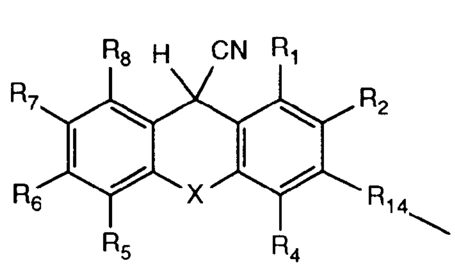

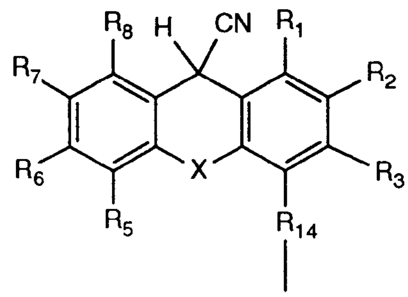 (V)

$R_{11}$ Hydroxy, $C_1$-$C_{12}$-Alkoxy oder

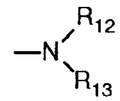

darstellt,
$R_{12}$ und $R_{13}$ unabhängig voneinander Wasserstoff oder $C_1$-$C_{12}$-Alkyl bedeuten,
$R_{14}$ eine direkte Bindung, $C_1$-$C_{12}$-Alkylen, durch Sauerstoff, Schwefel oder >N-$R_{10}$ unterbrochenes $C_2$-$C_{12}$-Alkylen;

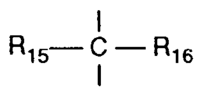 oder 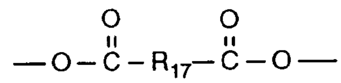

darstellt,
$R_{15}$ und $R_{16}$ unabhängig voneinander Wasserstoff, $C_1$-$C_8$-Alkyl oder Phenyl bedeuten,
oder $R_{15}$ und $R_{16}$ zusammen mit dem C-Atom, an das sie gebunden sind, einen unsubstituierten oder durch 1 bis 3 $C_1$-$C_4$-Alkyl substituierten $C_5$-$C_7$-Cycloalkylidenring bilden,
$R_{17}$ eine direkte Bindung, $C_1$-$C_{12}$-Alkylen, durch Sauerstoff, Schwefel oder >N-$R_{10}$ unterbrochenes $C_2$-$C_{12}$-Alkylen; $C_2$-$C_{12}$-Alkenylen, $C_2$-$C_{16}$-Alkyliden, $C_7$-$C_{12}$-Phenylalkyliden, $C_5$-$C_7$-Cycloalkylen, unsubstituiertes oder durch $C_1$-$C_4$-Alkyl substituiertes Phenylen, oder

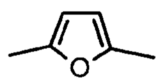

darstellt,
$R_{18}$ Wasserstoff, $C_1$-$C_{18}$-Alkyl, durch Sauerstoff, Schwefel oder >N-$R_{10}$ unterbrochenes $C_2$-$C_{18}$-Alkyl; unsubstituiertes oder durch $C_1$-$C_4$-Alkyl substituiertes $C_5$-$C_7$-Cycloalkyl; unsubstituiertes oder durch $C_1$-$C_4$-Alkyl substituiertes Phenyl; $C_7$-$C_9$-Phenylalkyl, Hydroxy, $C_1$-$C_{12}$-Alkoxy, durch Sauerstoff, Schwefel oder >N-$R_{10}$ unterbrochenes $C_3$-$C_{12}$-Alkoxy; $C_1$-$C_{18}$-Alkanoyl, durch Sauerstoff, Schwefel oder >N-$R_{10}$ unterbrochenes $C_3$-$C_{18}$-Alkanoyl; $C_3$-$C_{18}$-Alkenoyl, Benzoyl oder durch $C_1$-$C_8$-Alkyl substituiertes Benzoyl; Cyano, $-(CH_2)_mCOR_{11}$ oder

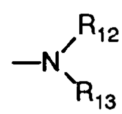

bedeutet, und
$R_{19}$ $C_1$-$C_{18}$-Alkyl, Benzyl oder $-CH_2CH_2OH$ darstellt.

Bevorzugt sind Zusammensetzungen enthaltend Verbindungen der Formel I, worin $R_5$, $R_5$, $R_7$ und $R_8$ Wasserstoff bedeuten.

Bevorzugt sind auch Zusammensetzungen enthaltend Verbindungen der Formel I, worin X eine direkte Bindung, Sauerstoff, Schwefel, $-CH_2CH_2-$, $-CH=CH-$, >N-$R_{18}$ oder

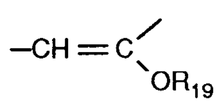

bedeutet,
$R_{18}$ $C_1$-$C_4$-Alkyl ist, und
$R_{19}$ $C_1$-$C_8$-Alkyl oder $-CH_2CH_2OH$ darstellt.

Bevorzugt sind ebenfalls Zusammensetzungen enthaltend Verbindungen der Formel I, worin
$R_1$, $R_2$, $R_3$ und $R_4$ unabhängig voneinander Wasserstoff, Chlor, $C_1$-$C_{18}$-Alkyl, durch Sauerstoff unterbrochenes $C_2$-$C_{18}$-Alkyl; $C_5$-$C_8$-Cycloalkyl, Phenyl, $C_7$-$C_9$-Phenylalkyl, Hydroxy, $C_1$-$C_{18}$-Alkoxy, durch Sauerstoff unter-

brochenes $C_3$-$C_{18}$-Alkoxy; $C_1$-$C_{18}$-Alkylthio, $C_1$-$C_{18}$-Alkanoyloxy, durch Sauerstoff unterbrochenes $C_3$-$C_{18}$-Alkanoyloxy; $C_3$-$C_{18}$-Alkenoyloxy, Benzoyloxy, Cyano, $-(CH_2)_mCOR_{11}$ oder

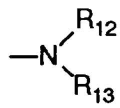

bedeuten, mit der Bedingung, dass mindestens einer der Reste $R_1$, $R_2$, $R_3$ oder $R_4$ Wasserstoff darstellt, $R_2$ zusätzlich einen Rest der Formel III und $R_4$ zusätzlich einen Rest der Formel V bedeutet, wobei gleichzeitig nur ein Rest der Formel m oder V in der Verbindung der Formel I auftritt,

(III) 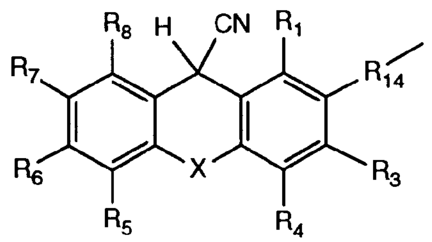 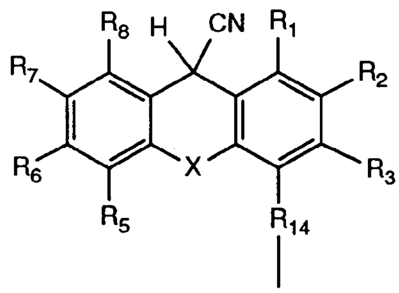 (V)

$R_5$, $R_6$, $R_7$ und $R_6$ unabhängig voneinander Wasserstoff, Chlor, $C_1$-$C_{18}$-Alkyl, $C_5$-$C_8$-Cycloalkyl, Phenyl, Benzyl, Hydroxy, $C_1$-$C_{18}$-Alkoxy, $C_1$-$C_{18}$-Alkylthio, $C_1$-$C_{18}$-Alkanoyloxy, $C_3$-$C_{18}$-Alkenoyloxy, Benzoyloxy, Cyano, $-(CH_2)_mCOR_{11}$ oder

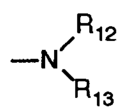

bedeuten, mit der Bedingung, dass mindestens einer der Reste $R_6$, $R_6$, $R_7$ oder $R_6$ Wasserstoff darstellt,
$R_{11}$ $C_1$-$C_{12}$-Alkoxy oder

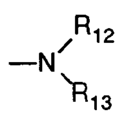

bedeutet,
$R_{12}$ und $R_{13}$ unabhängig voneinander Wasserstoff oder $C_1$-$C_8$-Alkyl darstellen,
$R_{14}$ $C_1$-$C_8$-Alkylen, durch Sauerstoff unterbrochenes $C_2$-$C_{12}$-Alkylen;

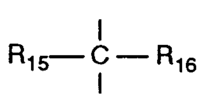

oder

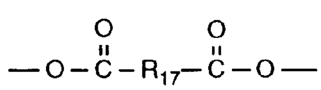

bedeutet,
$R_{15}$ und $R_{16}$ unabhängig voneinander Wasserstoff, $C_1$-$C_8$-Alkyl oder Phenyl darstellen, oder $R_{15}$ und $R_{16}$ zusammen mit dem C-Atom, an das sie gebunden sind, einen $C_5$-$C_7$-Cycloalkylidenring bilden, und
$R_{17}$ $C_1$-$C_8$-Alkylen, durch Sauerstoff unterbrochenes $C_2$-$C_8$-Alkylen; $C_2$-$C_8$-Alkenylen, $C_2$-$C_8$-Alkyliden, $C_5$-$C_7$-

Cycloalkylen oder Phenylen bedeutet.

Von besonderem Interesse sind Zusammensetzungen enthaltend Verbindungen der Formel I, worin $R_1$, $R_2$, $R_3$ und $R_4$ unabhängig voneinander Wasserstoff, $C_1$-$C_{18}$-Alkyl, $C_5$-$C_8$-Cycloalkyl, Phenyl, $C_7$-$C_9$-Phenylalkyl, Hydroxy, $C_1$-$C_{18}$-Alkoxy, $C_1$-$C_{18}$-Alkanoyloxy, $C_3$-$C_{18}$-Alkenoyloxy, Benzoyloxy, Cyano oder $-(CH_2)_mCOR_{11}$ bedeuten, mit der Bedingung, dass mindestens einer der Reste $R_1$, $R_2$, $R_3$ oder $R_4$ Wasserstoff darstellt, $R_2$ zusätzlich einen Rest der Formel III und $R_4$ zusätzlich einen Rest der Formel V bedeutet, wobei gleichzeitig nur ein Rest der Formel III oder V in der Verbindung der Formel I auftritt,

(III) 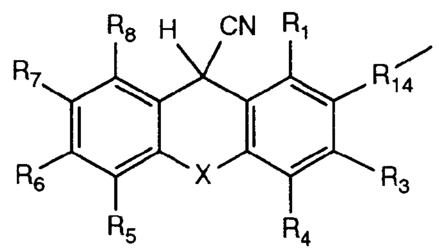 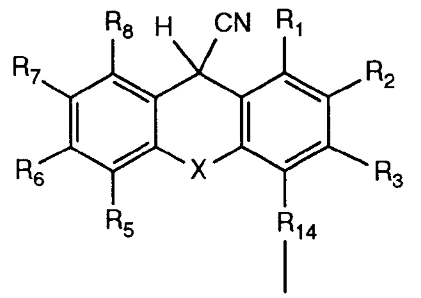 (V)

$R_5$, $R_6$, $R_7$ und $R_6$ unabhängig voneinander Wasserstoff, Chlor, $C_1$-$C_{18}$-Alkyl, $C_5$-$C_8$-Cycloalkyl, Phenyl, Benzyl oder Cyano bedeuten, mit der Bedingung, dass mindestens einer der Reste $R_6$, $R_6$, $R_7$ oder $R_6$ Wasserstoff darstellt,

$R_{11}$ $C_1$-$C_{12}$-Alkoxy ist,

$R_{14}$ $C_1$-$C_8$-Alkylen, durch Sauerstoff unterbrochenes $C_2$-$C_{12}$-Alkylen oder

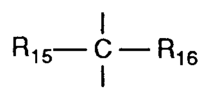

bedeutet,

$R_{15}$ und $R_{16}$ unabhängig voneinander Wasserstoff, $C_1$-$C_8$-Alkyl oder Phenyl darstellen, oder $R_{15}$ und $R_{16}$ zusammen mit dem C-Atom, an das sie gebunden sind, einen $C_5$-$C_7$-Cycloalkylidenring bilden,

$R_{18}$ Wasserstoff, $C_1$-$C_{12}$-Alkyl, Benzyl, Hydroxy, $C_1$-$C_{12}$-Alkoxy, $C_1$-$C_{18}$-Alkanoyl oder Benzoyl bedeutet,

X eine direkte Bindung, Sauerstoff, Schwefel, $-CH_2CH_2-$, -CH=CH- oder $>N-R_{18}$ darstellt, und

m 0 oder 1 ist.

Besonders bevorzugt sind Zusammensetzungen enthaltend Verbindungen der Formel I, worin $R_1$, $R_2$, $R_3$ und $R_4$ unabhängig voneinander Wasserstoff, $C_1$-$C_{12}$-Alkyl, Cyclohexyl, $C_7$-$C_9$-Phenylalkyl, Hydroxy, $C_1$-$C_{12}$-Alkoxy, $C_1$-$C_{12}$-Alkanoyloxy oder $-(CH_2)_mCOR_{11}$ bedeuten, mit der Bedingung, dass mindestens einer der Reste $R_1$, $R_2$, $R_3$ oder $R_4$ Wasserstoff darstellt,

$R_5$, $R_6$, $R_7$ und $R_6$ unabhängig voneinander Wasserstoff, Chlor, $C_1$-$C_8$-Alkyl oder Cyclohexyl bedeuten, mit der Bedingung, dass mindestens einer der Reste $R_6$, $R_6$, $R_7$ oder $R_8$ Wasserstoff darstellt,

$R_{11}$ $C_1$-$C_{12}$-Alkoxy ist,

$R_{18}$ Wasserstoff, $C_1$-$C_8$-Alkyl, Hydroxy, $C_1$-$C_6$-Alkanoyl oder Benzoyl bedeutet,

X eine direkte Bindung, Sauerstoff, Schwefel, $-CH_2CH_2-$, -CH=CH- oder $>N-R_{18}$ darstellt, und

m 0 oder 1 ist.

Speziell bevorzugt sind Zusammensetzungen enthaltend Verbindungen der Formel I, worin

$R_1$ Wasserstoff, Hydroxy oder $C_1$-$C_{12}$-Alkanoyloxy bedeutet,

$R_2$ Wasserstoff, $C_1$-$C_{12}$-Alkyl, $C_7$-$C_9$-Phenylalkyl, $C_1$-$C_3$-Alkoxy oder $-(CH_2)_mCOR_{11}$ darstellt,

$R_3$ Wasserstoff, Hydroxy oder $C_1$-$C_{12}$-Alkanoyloxy bedeutet,

$R_4$ Wasserstoff, $C_1$-$C_4$-Alkyl oder Cyclohexyl darstellt, mit der Bedingung, dass mindestens einer der Reste $R_1$, $R_2$, $R_3$ oder $R_4$ Wasserstoff ist,

$R_5$, $R_6$, $R_7$ und $R_6$ Wasserstoff bedeuten,

$R_{11}$ $C_1$-$C_{12}$-Alkoxy ist,

$R_{18}$ $C_1$-$C_4$-Alkyl darstellt,

X eine direkte Bindung, Sauerstoff, Schwefel, $-CH_2CH_2-$, -CH=CH- oder $>N-R_{18}$ bedeutet, und

m 0 oder 1 ist.

Die Verbindungen der Formel I eignen sich zum Stabilisieren von organischen Materialien gegen thermischen, oxidativen oder lichtinduzierten Abbau.

Beispiele für derartige Materialien sind:

1. Polymere von Mono- und Diolefinen, beispielsweise Polypropylen, Polyisobutylen, Polybuten-1, Poly-4-methylpenten-1, Polyisopren oder Polybutadien sowie Polymerisate von Cycloolefinen wie z.B. von Cyclopenten oder Norbornen; ferner Polyethylen (das gegebenenfalls vernetzt sein kann), z.B. Polyethylen hoher Dichte (HDPE), Polyethylen niederer Dichte (LDPE), lineares Polyethylen niederer Dichte (LLDPE), verzweigtes Polyethylen niederer Dichte (VLDPE).

Polyolefine, d.h. Polymere von Monoolefinen, wie sie beispielhaft im vorstehenden Absatz erwähnt sind, insbesondere Polyethylen und Polypropylen, können nach verschiedenen Verfahren hergestellt werden, insbesondere nach den folgenden Methoden:

a) radikalisch (gewöhnlich bei hohem Druck und hoher Temperatur).

b) mittels Katalysator, wobei der Katalysator gewöhnlich ein oder mehrere Metalle der Gruppe IVb, Vb, VIb oder VIII enthält. Diese Metalle besitzen gewöhnlich einen oder mehrere Liganden wie Oxide, Halogenide, Alkoholate, Ester, Ether, Amine, Alkyle, Alkenyle und/oder Aryle, die entweder $\pi$- oder $\sigma$-koordiniert sein können. Diese Metallkomplexe können frei oder auf Träger fixiert sein, wie beispielsweise auf aktiviertem Magnesiumchlorid, Titan(III)chlorid, Aluminiumoxid oder Siliziumoxid. Diese Katalysatoren können im Polymerisationsmedium löslich oder unlöslich sein. Die Katalysatoren können als solche in der Polymerisation aktiv sein, oder es können weitere Aktivatoren verwendet werden, wie beispielsweise Metallalkyle, Metallhydride, Metallalkylhalogenide, Metallalkyloxide oder Metallalkyloxane, wobei die Metalle Elemente der Gruppen Ia, IIa und/oder IIIa sind. Die Aktivatoren können beipielsweise mit weiteren Ester-, Ether-, Amin- oder Silylether-Gruppen modifiziert sein. Diese Katalysatorsysteme werden gewöhnlich als Phillips, Standard Oil Indiana, Ziegler (-Natta), TNZ (DuPont), Metallocen oder Single Site Katalysatoren (SSC) bezeichnet.

2. Mischungen der unter 1) genannten Polymeren, z.B. Mischungen von Polypropylen mit Polyisobutylen, Polypropylen mit Polyethylen (z.B. PP/HDPE, PP/LDPE) und Mischungen verschiedener Polyethylentypen (z.B. LDPE/HDPE).

3. Copolymere von Mono- und Diolefinen untereinander oder mit anderen Vinylmonomeren, wie z.B. Ethylen-Propylen-Copolymere, lineares Polyethylen niederer Dichte (LLDPE) und Mischungen desselben mit Polyethylen niederer Dichte (LDPE), Propylen-Buten-1-Copolymere, Propylen-Isobutylen-Copolymere, Ethylen-Buten-1-Copolymere, Ethylen-Hexen-Copolymere, Ethylen-Methylpenten-Copolymere, Ethylen-Hepten-Copolymere, Ethylen-Octen-Copolymere, Propylen-Butadien-Copolymere, Isobutylen-Isopren-Copolymere, Ethylen-Alkylacrylat-Copolymere, Ethylen-Alkylmethacrylat- Copolymere, Ethylen-Vinylacetat-Copolymere und deren Copolymere mit Kohlenstoffmonoxid, oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen; ferner Mischungen solcher Copolymere untereinander und mit unter 1) genannten Polymeren, z.B. Polypropylen/Ethylen-Propylen-Copolymere, LDPE/Ethylen-Vinylacetat-Copolymere, LDPE/Ethylen-Acrylsäure-Copolymere, LLDPE/Ethylen-Vinylacetat-Copolymere, LLDPE/Ethylen-Acrylsäure-Copolymere und alternierend oder statistisch aufgebaute Polyalkylen/Kohlenstoffmonoxid-Copolymere und deren Mischungen mit anderen Polymeren wie z.B. Polyamiden.

4. Kohlenwasserstoffharze (z.B. $C_5$-$C_9$) inklusive hydrierte Modifikationen davon (z.B. Klebrigmacherharze) und Mischungen von Polyalkylenen und Stärke.

5. Polystyrol, Poly-(p-methylstyrol), Poly-($\alpha$-methylstyrol).

6. Copolymere von Styrol oder $\alpha$-Methylstyrol mit Dienen oder Acrylderivaten, wie z.B. Styrol-Butadien, Styrol-Acrylnitril, Styrol-Alkylmethacrylat, Styrol-Butadien-Alkylacrylat und -methacrylat, Styrol-Maleinsäureanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

7. Pfropfcopolymere von Styrol oder $\alpha$-Methylstyrol, wie z.B. Styrol auf Polybutadien, Styrol auf Polybutadien-Styrol- oder Polybutadien-Acrylnitril-Copolymere, Styrol und Acrylnitril (bzw. Methacrylnitril) auf Polybutadien; Styrol, Acrylnitril und Methylmethacrylat auf Polybutadien; Styrol und Maleinsäureanhydrid auf Polybutadien; Styrol, Acrylnitril und Maleinsäureanhydrid oder Maleinsäureimid auf Polybutadien; Styrol und Maleinsäureimid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 6) genannten Copolymeren, wie sie z.B. als sogenannte ABS-, MBS-, ASA- oder AES-Polymere bekannt sind.

8. Halogenhaltige Polymere, wie z.B. Polychloropren, Chlorkautschuk, chloriertes oder chlorsulfoniertes

Polyethylen, Copolymere von Ethylen und chloriertem Ethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.

9. Polymere, die sich von $\alpha,\beta$-ungesättigten Säuren und deren Derivaten ableiten, wie Polyacrylate und Polymethacrylate, mit Butylacrylat schlagzäh modifizierte Polymethylmethacrylate, Polyacrylamide und Polyacrylnitrile.

10. Copolymere der unter 9) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-VinylhalogenidCopolymere oder Acrylnitril-Alkylmethacrylat-Butadien-Terpolymere.

11. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin; sowie deren Copolymere mit in Punkt 1 genannten Olefinen.

12. Homo- und Copolymere von cyclischen Ethern, wie Polyalkylenglykole, Polyethylenoxyd, Polypropylenoxyd oder deren Copolymere mit Bisglycidylethern.

13. Polyacetale, wie Polyoxymethylen, sowie solche Polyoxymethylene, die Comonomere, wie z.B. Ethylenoxid, enthalten; Polyacetale, die mit thermoplastischen Polyurethanen, Acrylaten oder MBS modifiziert sind.

14. Polyphenylenoxide und -sulfide und deren Mischungen mit Styrolpolymeren oder Polyamiden.

15. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.

16. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, 6/10, 6/9, 6/12, 4/6, 12/12, Polyamid 11, Polyamid 12, aromatische Polyamide ausgehend von m-Xylol, Diamin und Adipinsäure; Polyamide, hergestellt aus Hexamethylendiamin und Iso- und/oder Terephthalsäure und gegebenenfalls einem Elastomer als Modifikator, z.B. Poly-2,4,4-trimethylhexamethylenterephthalamid oder Poly-m-phenylen-isophthalamid. Block-Copolymere der vorstehend genanntenPolyamide mit Polyolefinen, Olefin-Copolymeren, Ionomeren oder chemisch gebundenen oder gepfropften Elastomeren; oder mit Polyethern, wie z.B. mit Polyethylenglykol, Polypropylenglykol oder Polytetramethylenglykol. Ferner mit EPDM oder ABS modifizierte Polyamide oder Copolyamide; sowie während der Verarbeitung kondensierte Polyamide ("RIM-Polyamidsysteme").

17. Polyharnstoffe, Polyimide, Polyamid-imide und Polybenzimidazole.

18. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten; ferner mit Polycarbonaten oder MBS modifizierte Polyester.

19. Polycarbonate und Polyestercarbonate.

20. Polysulfone, Polyethersulfone und Polyetherketone.

21. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

22. Trocknende und nicht-trocknende Alkydharze.

23. Ungesättigte Polyesterharze, die sich von Copolyestern gsättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

24. Vernetzbare Acrylharze, die sich von substituierten Acrylsäureestern ableiten, wie z.B. von Epoxyacrylaten, Urethan-acrylaten oder Polyester-acrylaten.

25. Alkydharze, Polyesterharze und Acrylatharze, die mit Melaminharzen, Harnstoffharzen, Polyisocyanaten oder Epoxidharzen vernetzt sind.

26. Vernetzte Epoxidharze, die sich von Polyepoxiden ableiten, z.B. von Bis-glycidylethern oder von cycloaliphatischen Diepoxiden.

27. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose; sowie Kolophoniumharze und Derivate.

28. Mischungen (Polyblends) der vorgenannten Polymeren, wie z.B. PP/EPDM, Polyamid/EPDM oder ABS, PVC/EVA, PVC/ABS, PVC/MBS, PC/ABS, PBTP/ABS, PC/ASA, PC/PBT, PVC/CPE, PVC/Acrylate, POM/thermoplastisches PUR, PC/thermoplastisches PUR, POM/Acrylat, POM/MBS, PPO/HIPS,

PPO/PA 6.6 und Copolymere, PA/HDPE, PA/PP, PA/PPO.

29. Natürliche und synthetische organische Stoffe, die reine monomere Verbindungen oder Mischungen von solchen darstellen, beispielsweise Mineralöle, tierische oder pflanzliche Fette, Oele und Wachse, oder Oele, Wachse und Fette auf Basis synthetischer Ester (z.B. Phthalate, Adipate, Phosphate oder Trimellitate), sowie Abmischungen synthetischer Ester mit Mineralölen in beliebigen Gewichtsverhältnissen, wie sie z.B. als Spinnpräparationen Anwendung finden, sowie deren wässrige Emulsionen.

30. Wässrige Emulsionen natürlicher oder synthetischer Kautschuke, wie z.B. Naturkautschuk-Latex oder Latices von carboxylierten Styrol-Butadien-Copolymeren.

Bevorzugte organische Materialien sind Polymere, z.B. natürliche, halbsynthetische oder synthetische Polymere, insbesondere thermoplastische Polymere, Tackifiers oder Klebstoffe. Besonders bevorzugt sind Polyolefine, z.B. Polypropylen oder Polyethylen.

Besonders hervorzuheben ist die Wirkung der erfindungsgemässen Verbindungen gegen thermischen und oxidativen Abbau, vor allem bei thermischer Belastung, wie sie bei der Verarbeitung von Thermoplasten auftritt. Die erfindungsgemässen Verbindungen sind daher hervorragend als Verarbeitungsstabilisatoren einzusetzen.

Vorzugsweise werden die Verbindungen der Formel I dem zu stabilisierenden Material in Mengen von 0,0005 bis 5 %, insbesondere 0,001 bis 2 %, beispielsweise 0,01 bis 2 %, zugesetzt, bezogen auf das Gewicht des zu stabilisierenden organischen Materials.

Zusätzlich zu den Verbindungen der Formel I können die erfindungsgemässen Zusammensetzungen weitere Costabilisatoren enthalten, wie beispielsweise die folgenden:

1. Antioxidantien

1.1. Alkylierte Monophenole, z.B. 2,6-Di-tert-butyl-4-methylphenol, 2-butyl-4,6-dimethylphenol, 2,6-Di-tert-butyl-4-ethylphenol, 2,6-Di-tert-butyl-4-n-butylphenol, 2,6-Di-tert-butyl-4-iso-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tri-cyclohexylphenol, 2,6-Di-tert-butyl-4-methoxymethylphenol, 2,6-Di-nonyl-4-methylphenol, 2,4-Dimethyl-6-(1'-methyl-undec-1'-yl)-phenol, 2,4-Dimethyl-6-(1'-methyl-heptadec-1'-yl)-phenol, 2,4-Dimethyl-6-(1'-methyl-tridec-1'-yl)-phenol und Mischungen davon.

1.2. Alkylthiomethylphenole, z.B. 2,4-Di-octylthiomethyl-6-tert-butylphenol, 2,4-Di-octylthiomethyl-6-methylphenol, 2,4-Di-octylthiomethyl-6-ethylphenol, 2,6-Di-dodecylthiomethyl-4-nonylphenol.

1.3. Hydrochinone und alkylierte Hydrochinone, z.B. 2,6-Di-tert-butyl-4-methoxyphenol, 2,5-Di-tert-butyl-hydrochinon, 2,5-Di-tert-amyl-hydrochinon, 2,6-Diphenyl-4-octadecyloxyphenol, 2,6-Di-tert-butyl-hydrochinon, 2,5-Di-tert-butyl-4-hydroxyanisol, 3,5-Di-tertbutyl-4-hydroxyanisol, 3,5-Di-tert-butyl-4-hydroxyphenyl-stearat, Bis-(3,5-di-tert-butyl-4-hydroxyphenyl)adipat.

1.4. Hydroxylierte Thiodiphenylether, z.B. 2,2'-Thio-bis-(6-tert-butyl-4-methylphenol), 2,2'-Thio-bis-(4-octylphenol), 4,4'-Thio-bis-(6-tert-butyl-3-methylphenol), 4,4'-Thio-bis-(6-tert-butyl-2-methylphenol), 4,4'-Thio-bis-(3,6-di-sec.-amylphenol), 4,4'-Bis-(2,6-di-methyl-4-hydroxyphenyl)-disulfid.

1.5. Alkyliden-Bisphenole, z.B. 2,2'-Methylen-bis-(6-tert-butyl-4-methylphenol), 2,2'-Methylen-bis-(6-tert-butyl-4-ethylphenol), 2,2'-Methylen-bis-[4-methyl-6-(α-methylcyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-tert-butylphenol), 2,2'-Ethyliden-bis-(4,6-di-tert-butylphenol), 2,2'-Ethyliden-bis-(6-tert-butyl-4-isobutylphenol), 2,2'-Methylen-bis-[6-(α-methylbenzyl)-4-nonylphenol], 2,2'-Methylen-bis-[6-(α,α-di-methylbenzyl)-4-nonylphenol], 4,4'-Methylen-bis-(2,6-di-tert-butylphenol), 4,4'-Methylen-bis-(6-tert-butyl-2-methylphenol), 1,1-Bis-(5-tert-butyl-4-hydroxy-2-methylphenyl)-butan, 2,6-Bis-(3-tert-butyl-5-methyl-2-hydroxybenzyl)-4-methylphenol, 1,1,3-Tris-(5-tert-butyl-4-hydroxy-2-methylphenyl)-butan, 1,1-Bis-(5-tert-butyl-4-hydroxy-2-methylphenyl)-3-n-dodecylmercaptobutan, Ethylenglycol-bis-[3,3-bis-(3'-tert-butyl-4'-hydroxyphenyl)-butyrat], Bis-(3-tert-butyl-4-hydroxy-5-methyl-phenyl)-dicyclopentadien, Bis-[2-(3'-tert-butyl-2'-hydroxy-5'-methyl-benzyl)-6-tert-butyl-4-methyl-phenyl]-terephthalat, 1,1-Bis-(3,5-dimethyl-2-hydroxyphenyl)-butan, 2,2-Bis-(3,5-di-tert-butyl-4-hydroxyphenyl)-propan, 2,2-Bis-(5-tert-butyl-4-hydroxy-2-methylphenyl)-4-n-dodecylmercaptobutan, 1,1,5,5-Tetra-(5-tert-butyl-4-hydroxy-2-methylphenyl)-pentan.

1.6. O-, N- und S-Benzylverbindungen, z.B. 3,5,3',5'-Tetra-tert-butyl-4,4'-dihydroxydibenzylether, Octadecyl-4-hydroxy-3,5-dimethylbenzyl-mercaptoacetat, Tris-(3,5-di-tert-butyl-4-hydroxybenzyl)-amin, Bis-(4-tert-butyl-3-hydroxy-2,6-dimethylbenzyl)-di-thioterephthalat, Bis-(3,5-di-tert-butyl-4-hydroxybenzyl)-sulfid, Isooctyl-3,5-di-tert-butyl-4-hydroxybenzyl-mercaptoacetat.

1.7. Hydroxybenzylierte Malonate, z.B. Dioctadecyl-2,2-bis-(3,5-di-tert-butyl-2-hydroxybenzyl)-malonat, Di-octadecyl-2-(3-tert-butyl-4-hydroxy-5-methylbenzyl)-malonat, Di-dodecylmercaptoethyl-2,2-bis-(3,5-di-tert-butyl-4-hydroxybenzyl)-malonat, Di-[4-(1,1,3,3-tetramethylbutyl)-phenyl]-2,2-bis-(3,5-di-tert-butyl-4-hydroxybenzyl)-malonat.

1.8. Hydroxybenzyl-Aromaten, z.B. 1,3,5-Tris-(3,5-di-tert-butyl-4-hydroxybenzyl)-2,4,6-trimethylbenzol, 1,4-Bis-(3,5-di-tert-butyl-4-hydroxybenzyl)-2,3,5,6-tetramethylbenzol, 2,4,6-Tris-(3,5-di-tert-butyl-4-hydroxybenzyl)-phenol.

1.9. Triazinverbindungen, z.B. 2,4-Bis-octylmercapto-6-(3,5-di-tert-butyl-4-hydroxyanilino)-1,3,5-triazin, 2-Octylmercapto-4,6-bis-(3,5-di-tert-butyl-4-hydroxyanilino)-1,3,5-triazin, 2-Octylmercapto-4,6-bis-(3,5-di-tert-butyl-4-hydroxyphenoxy)-1,3,5-triazin, 2,4,6-Tris-(3,5-di-tert-butyl-4-hydroxyphenoxy)-1,2,3-triazin, 1,3,5-Tris-(3,5-di-tert-butyl-4-hydroxybenzyl)-isocyanurat, 1,3,5-Tris-(4-tert-butyl-3-hydroxy-2,6-dimethylbenzyl)-isocyanurat, 2,4,6-Tris-(3,5-di-tert-butyl-4-hydroxyphenylethyl)-1,3,5-triazin, 1,3,5-Tris-(3,5-di-tert-butyl-4-hydroxyphenylpropionyl)-hexahydro-1,3,5-triazin, 1,3,5-Tris-(3,5-dicyclohexyl-4-hydroxybenzyl)-isocyanurat.

1.10. Benzylphosphonate, z.B. Dimethyl-2,5-di-tert-butyl-4-hydroxybenzylphosphonat, Diethyl-3,5-di-tert-butyl-4-hydroxybenzylphosphonat, Dioctadecyl-3,5-di-tert-butyl-4-hydroxybenzylphosphonat, Dioctadecyl-5-tert-butyl-4-hydroxy-3-methylbenzylphosphonat, Ca-Salz des 3,5-Di-tert-butyl-4-hydroxybenzyl- phosphonsäure-monoethylesters.

1.11. Acylaminophenole, z.B. 4-Hydroxy-laurinsäureanilid, 4-Hydroxystearinsäureanilid, N-(3,5-di-tert-butyl-4-hydroxyphenyl)-carbaminsäureoctylester.

1.12. Ester der β-(3,5-Di-tert-butyl-4-hydroxyphenyl)-propionsäure mit ein- oder mehr wertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxyethyl)-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.13. Ester der β-(5-tert-Butyl-4-hydroxy-3-methylphenyl)-propionsäure mit ein- oder mehrwertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.14. Ester der β-(3,5-Dicyclohexyl-4-hydroxyphenyl)-propionsäure mit ein- oder mehr wertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.15. Ester der 3,5-Di-tert-butyl-4-hydroxyphenylessigsäure mit ein- oder mehrwertigen Alkoholen, wie z.B. mit Methanol, Ethanol, Octadecanol, 1,6-Hexandiol, 1,9-Nonandiol, Ethylenglycol, 1,2-Propandiol, Neopentylglycol, Thiodiethylenglycol, Diethylenglycol, Triethylenglycol, Pentaerythrit, Tris-(hydroxy)ethyl-isocyanurat, N,N'-Bis-(hydroxyethyl)-oxalsäurediamid, 3-Thiaundecanol, 3-Thiapentadecanol, Trimethylhexandiol, Trimethylolpropan, 4-Hydroxymethyl-1-phospha-2,6,7-trioxabicyclo-[2.2.2]-octan.

1.16. Amide der β-(3,5-Di-tert-butyl-4-hydroxyphenyl)-propionsäure, wie z.B. N,N'-Bis-(3,5-di-tert-butyl-4-hydroxyphenylpropionyl)-hexamethylendiamin, N,N'-Bis-(3,5-di-tert-butyl-4-hydroxyphenylpropionyl)-trimethylendiamin, N,N'-Bis-(3,5-di-tert-butyl-4-hydroxyphenylpropionyl)-hydrazin.

2. UV-Absorber und Lichtschutzmittel

2.1. 2-(2'-Hydroxyphenyl)-benzotriazole, wie z.B. 2-(2'-Hydroxy-5'-methylphenyl)-benzotriazol, 2-(3',5'-Di-tert-butyl-2'-hydroxyphenyl)-benzotriazol, 2-(5-tert-Butyl-2'-hydroxyphenyl)-benzotriazol, 2-(2'-Hydroxy-5'-(1,1,3,3-tetramethylbutyl)phenyl)-benzotriazol, 2-(3',5'-Di-tert-butyl-2'-hydroxyphenyl)- 5-chlor-benzotriazol, 2-(3'-tert-Butyl-2'-hydroxy-5'-methylphenyl)-5-chlor-benzotriazol, 2-(3'-sec-Butyl-5'-tert-butyl-2'-hydroxyphenyl)-benzotriazol, 2-(2'-Hydroxy-4'-octoxyphenyl)-benzotriazol, 2-(3',5'-Di-tert-amyl-2'-hydroxyphenyl)-benzotriazol, 2-(3',5'-Bis-(α,α-dimethylbenzyl)-2'-hydroxyphenyl)-benzotriazol, Mischung aus 2-(3'-tert-Butyl-2'-hydroxy-5'-(2-octyloxycarbonylethyl)-phenyl)-5-chlor-benzotriazol, 2-(3'-tert-Butyl-5'-[2-(2-ethylhexyloxy)-carbonylethyl]-2'-hydroxyphenyl)-5-chlor-benzotriazol, 2-(3'-tert-Butyl-2'-hydroxy-5'-(2-methoxycarbonylethyl)phenyl)-5-chlor-benzotriazol, 2-(3'-tert-Butyl-2'-hydroxy-5'-(2-methoxycarbonylethyl)phenyl)-benzotriazol, 2-(3'-tert-Butyl-2'-hydroxy-5'-(2-octyloxycarbonylethyl)phenyl)-benzotriazol, 2-(3'-tert-Butyl-5'-[2-(2-ethylhexyloxy)carbonylethyl]-2'-hydroxyphenyl)-benzotriazol, 2-(3'-Dodecyl-2'-hydroxy-5'-methylphenyl)-benzotriazol, und 2-(3'-tert-Butyl-2'-hydroxy-5'-(2-isooctyloxycarbonylethyl)phenyl-benzotriazol, 2,2'-Methylen-bis[4-(1,1,3,3-tetramethylbutyl)-6-benzotriazol-2-yl-phenol]; Umesterungsprodukt von 2-[3'-tert-Butyl-5'-(2-methoxycarbonylethyl)-2'-hydroxy-phenyl]-benzotriazol mit Polyethylenglycol 300; $[R-CH_2CH_2-COO(CH_2)_3]_2$ mit R = 3'-tert-Butyl-4'-hydroxy-5'-2H-benzotriazol-2-yl-phenyl.

2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

2.3. Ester von gegebenenfalls substituierten Benzoesäuren, wie z.B. 4-tert-Butyl-phenylsalicylat, Phenylsalicylat, Octylphenyl-salicylat, Dibenzoylresorcin, Bis-(4-tert-butylbenzoyl)-resorcin, Benzoylresorcin, 3,5-Di-tert-butyl-4-hydroxybenzoesäure-2,4-di-tert-butylphenylester, 3,5-Di-tert-butyl-4-hydroxybenzoesäurehexadecylester, 3,5-Di-tert-butyl-4-hydroxybenzoesäure-octadecylester, 3,5-Di-tert-butyl-4-hydroxybenzoesäure-2-methyl-4,6-di-tert-butylphenylester.

2.4. Acrylate, wie z.B. α-Cyan-β,β-diphenylacrylsäure-ethylester bzw. -isooctylester, α-Carbomethoxy-zimtsäuremethylester, α-Cyano-β-methyl-p-methoxy-zimtsäuremethyl-ester bzw. -butylester, α-Carbomethoxy-p-methoxy-zimtsäure-methylester, N-(β-Carbomethoxy-β-cyanovinyl)-2-methyl-indolin.

2.5. Nickelverbindungen, wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert-butylbenzylphosphonsäure-monoalkylestern, wie vom Methyl- oder Ethylester, Nickelkomplexe von Ketoximen, wie von 2-Hydroxy-4-methyl-phenyl-undecylketoxim, Nickelkomplexe des 1-Phenyl-4-lauroyl-5-hydroxy-pyrazols, gegebenenfalls mit zusätzlichen Liganden.

2.6. Sterisch gehinderte Amine, wie z.B. Bis-(2,2,6,6-tetramethyl-piperidyl)-sebacat, Bis-(2,2,6,6-tetramethyl-piperidyl)-succinat, Bis-(1,2,2,6,6-pentamethylpiperidyl)-sebacat, n-Butyl-3,5-di-tert-butyl-4-hydroxybenzyl-malonsäure-bis(1,2,2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-Bis-(2,2,6,6-Tetramethyl-4-piperidyl)-hexamethylendiamin und 4-tert-Octylamino-2,6-dichlor-1,3,5-s-triazin, Tris-(2,2,6,6-tetramethyl- 4-piperidyl)-nitrilotriacetat, Tetrakis-(2,2,6,6-tetramethyl-4-piperidyl)-1,2,3,4-butantetraoat, 1,1'-(1,2-Ethandiyl)-bis-(3,3,5,5-tetramethyl-piperazinon), 4-Benzoyl-2,2,6,6-tetramethylpiperidin,4-Stearyloxy-2,2,6,6-tetramethylpiperidin, Bis-(1,2,2,6,6-pentamethylpiperidyl)-2-n-butyl-2-(2-hydroxy-3,5-di-tert-butylbenzyl)-malonat, 3-n-Octyl-7,7,9,9-tetramethyl-1,3,8-triazaspiro[4.5]decan-2,4-dion, Bis-(1-octyloxy-2,2,6,6-tetramethylpiperidyl)-sebacat, Bis-(1-octyloxy-2,2,6,6-tetramethylpiperidyl)-succinat, Kondensationsprodukt aus N,N'-Bis-(2,2,6,6-tetramethyl-4-piperidyl)-hexamethylendiamin und 4-Morpholino-2,6-dichlor-1,3,5-triazin, Kondensationsprodukt aus 2-Chlor-4,6-di-(4-n-butylamino-2,2,6,6-tetramethylpiperidyl)-1,3,5-triazin und 1,2-Bis-(3-aminopropylamino)äthan, Kondensationsprodukt aus 2-Chlor-4,6-di-(4-n-butylamino-1,2,2,6,6-pentamethylpiperidyl)-1,3,5-triazin und 1,2-Bis-(3-aminopropylamino)-äthan, 8-Acetyl-3-dodecyl-7,7,9,9-tetramethyl-1,3,8-triazaspiro-[4.5]decan-2,4-dion, 3-Dodecyl-1-(2,2,6,6-tetramethyl-4-piperidyl)pyrrolidin-2,5-dion, 3-Dodecyl-1-(1,2,2,6,6-pentamethyl-4-piperidyl)-pyrrolidin-2,5-dion.

2.7. Oxalsäurediamide, wie z.B. 4,4'-Di-octyloxy-oxanilid, 2,2'-Di-octyloxy-5,5'-di-tert-butyl-oxanilid, 2,2'-Di-dodecyloxy-5,5'di-tert-butyl-oxanilid, 2-Ethoxy-2'-ethyl-oxanilid, N,N'-Bis-(3-dimethylaminopropyl)-oxalamid, 2-Ethoxy-5-tert-butyl-2'-ethyloxanilid und dessen Gemisch mit 2-Ethoxy-2'-ethyl-5,4'-di-tert-butyl-oxanilid, Gemische von o- und p-Methoxy- sowie von o- und p-Ethoxy-di-substituierten Oxaniliden.

2.8. 2-(2-Hydroxyphenyl)-1,1,3,5-triazine, wie z.B. 2,4,6-Tris(2-hydroxy-4-octyloxyphenyl)-1,3,5-triazin, 2-(2-Hydroxy-4-octyloxyphenyl)-4,6-bis-(2,4-dimethylphenyl)-1,3,5-triazin, 2-(2,4-Dihydroxyphenyl)-4,6-bis(2,4-dimethylphenyl)-1,3,5-triazin, 2,4-Bis-(2-hydroxy-4-propyloxyphenyl)-6-(2,4-dimethylphenyl)-1,3,5-triazin, 2-(2-Hydroxy-4-octyloxyphenyl)-4,6-bis(4-methylphenyl)-1,3,5-triazin, 2-(2-Hydroxy-4-dodecyloxyphenyl)-4,6-bis(2,4-dimethylphenyl)-1,3,5-triazin, 2-[2-hydroxy-4-(2-hydroxy-3-butyloxy-propyloxy)phenyl]-4,6-bis(2,4-dimethylphenyl)-1,3,5-triazin, 2-[2-hydroxy-4-(2-hydroxy-3-octyloxy-propyloxy)phenyl]-4,6-bis(2,4-dimethylphenyl)-1,3,5-triazin.

3. Metalldesaktivatoren, wie z.B. N,N'-Diphenyloxalsäurediamid, N-Salicylal-N'-salicyloylhydrazin, N,N'-Bis-(salicyloyl)-hydrazin, N,N'-Bis-(3,5-di-tert-butyl-4-hydroxyphenylpropionyl)-hydrazin, 3-Salicyloylamino-1,2,4-triazol, Bis-(benzyliden)-oxalsäuredihydrazid, Oxanilid, Isophthalsäure-dihydrazid, Sebacinsäure-bisphenylhydrazid, N,N'-Diacetyl-adipinsäure-dihydrazid, N,N'-Bis-salicyloyl-oxalsäure-dihydrazid, N,N'-Bis-salicyloyl-thiopropionsäure-dihydrazid.

4. Phosphite und Phosphonite, wie z.B. Triphenylphosphit, Diphenylalkylphosphite, Phenyldialkylphosphite, Tris-(nonylphenyl)-phosphit, Trilaurylphosphit, Trioctadecylphosphit, Distearyl-pentaerythritdiphosphit, Tris-(2,4-di-tert-butylphenyl)-phosphit, Diisodecylpentaerythrit-diphosphit, Bis-(2,4-di-tert-butylphenyl)-pentaerythritdiphosphit, Bis-(2,6-di-tert-butyl-4-methylphenyl)-pentaerythritdiphosphit, Bis-isodecyloxy-pentaerythritdiphosphit, Bis-(2,4-di-tert-butyl-6-methylphenyl)-pentaerythritdiphosphit, Bis-(2,4,6-tri-tert-butylphenyl)-pentaerythritdiphosphit, Tristearyl-sorbit-triphosphit, Tetrakis-(2,4-di-tert-butylphenyl)-4,4'-biphenylen-diphosphonit, 6-Isooctyloxy-2,4,8,10-tetra-tert-butyl-12H-dibenz[d,g]-1,3,2-dioxaphosphocin, 6-Fluor-2,4,8,10-tetra-tert-butyl-12-methyl-dibenz[d,g]-1,3,2-dioxaphosphocin, Bis-(2,4-di-tert-butyl-6-methylphenyl)-methylphosphit, Bis-(2,4-di-tert-butyl-6-methylphenyl)-ethylphosphit.

5. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.

6. Polyamidstabilisatoren, wie z.B. Kupfersalze in Kombination mit Jodiden und/oder Phosphorverbindungen und Salze des zweiwertigen Mangans.

7. Basische Co-Stabilisatoren, wie z.B. Melamin, Polyvinylpyrrolidon, Dicyandiamid, Tri-allylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Behenat, Mg-Stearat, Na-Ricinoleat, K-Palmitat, Antimonbrenzcatechinat oder Zinnbrenzcatechinat.

8. Nukleierungsmittel, wie z.B. 4-tert-Butylbenzoesäure, Adipinsäure, Diphenylessigsäure.

9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Ruß, Graphit.

10. Sonstige Zusätze, wie z.B. Weichmacher, Gleitmittel, Emulgatoren, Pigmente, Optische Aufheller, Flammschutzmittel, Antistatika, Treibmittel.

11. Benzofuranone bzw. Indolinone, wie z.B. in US-A-4 325 863, US-A-4 338 244 oder US-A-5 175 312 beschrieben, oder 3-[4-(2-Acetoxyethoxy)phenyl]-5,7-di-tert-butyl-benzofuran-2-on, 5,7-Di-tert-butyl-3-[4-(2-stearoyloxyethoxy)phenyl]-benzofuran-2-on, 3,3'-Bis-[5,7-di-tert-butyl-3-(4-[2-hydroxyethoxy]phenyl)-benzofuran-2-on], 5,7-Di-tert-butyl-3-(4-ethoxyphenyl)benzofuran-2-on, 3-(4-Acetoxy-3,5-dimethylphenyl)-5,7-di-tert-butyl-benzofuran-2-on, 3-(3,5-Dimethyl-4-pivaloyloxy-phenyl)-5,7-di-tert-butyl-benzofuran-2-on.

Die Costabilisatoren werden beispielsweise in Konzentrationen von 0,01 bis 10 %, bezogen auf das Gesamtgewicht des zu stabilisierenden Materials, zugesetzt.

Weitere bevorzugte Zusammensetzungen enthalten neben der Komponente (a) und den Verbindungen der Formel I zusätzlich noch weitere Additive, insbesondere phenolische Antioxidantien, Lichtschutzmittel und/oder Verarbeitungsstabilisatoren.

Besonders bevorzugte Additive sind phenolische Antioxidantien (Punkt 1 der Liste), sterisch gehinderte Amine (Punkt 2.6 der Liste), Phosphite und Phosphonite (Punkt 4 der Liste) und peroxidzerstörende Verbindungen (Punkt 5 der Liste).

Die Einarbeitung der Verbindungen der Formel I sowie gegebenenfalls weiterer Additive in das polymere, organische Material erfolgt nach bekannten Methoden, beispielsweise vor oder während der Formgebung oder auch durch Aufbringen der gelösten oder dispergierten Verbindungen auf das polymere, organische Material, gegebenenfalls unter nachträglichem Verdunsten des Lösungsmittels. Die Verbindungen der Formel I können auch in Form eines Masterbatches, der diese beispielsweise in einer Konzentration von 2,5 bis 25 Gew.-% enthält, den zu stabilisierenden Materialien zugesetzt werden.

Die Verbindungen der Formel I können auch vor oder während der Polymerisation oder vor der Vernetzung zugegeben werden.

Die Verbindungen der Formel I können in reiner Form oder in Wachsen, Oelen oder Polymeren verkapselt in das zu stabilisierende Material eingearbeitet werden.

Die Verbindungen der Formel I können auch auf das zu stabilisierende Polymer aufgesprüht werden. Sie sind in der Lage, andere Zusätze (z.B. die oben angegebenen herkömmlichen Additive) bzw. deren Schmelzen zu verdünnen, so dass sie auch zusammen mit diesen Zusätzen auf das zu stabilisierende Polymer aufgesprüht werden können. Besonders vorteilhaft ist die Zugabe durch Aufsprühen während der Desaktivierung der Polymerisationskatalysatoren, wobei z.B. der zur Desaktivierung verwendete Dampf zum Versprühen verwendet werden kann.

Bei kugelförmig polymerisierten Polyolefinen kann es z.B. vorteilhaft sein, die Verbindungen der Formel I, gegebenenfalls zusammen mit anderen Additiven, durch Aufsprühen zu applizieren.

Die so stabilisierten Materialien können in verschiedenster Form angewendet werden, z.B. als Folien, Fasern, Bändchen, Formmassen, Profile oder als Bindemittel für Lacke, Klebstoffe oder Kitte.

Die vorliegende Erfindung betrifft auch ein Verfahren zum Stabilisieren eines organischen Materials gegen oxidativen, thermischen oder lichtinduzierten Abbau, das dadurch gekennzeichnet ist, dass man diesem mindestens eine Verbindung der Formel I einverleibt oder auf dieses aufbringt.

Wie bereits hervorgehoben, werden die erfindungsgemässen Verbindungen besonders vorteilhaft als Stabilisatoren in Polyolefinen eingesetzt, vor allem als Thermostabilisatoren. Ausgezeichnete Stabilisierung

wird z.B. dann erhalten, wenn man sie in Kombination mit organischen Phosphiten oder Phosphoniten einsetzt. Dabei weisen die erfindungsgemässen Verbindungen den Vorteil auf, dass sie bereits in ausserordentlich geringen Mengen wirksam sind. Sie werden z.B. in Mengen von 0,0001 bis 0,015, insbesondere 0,0001 bis 0,008 Gew. % bezogen auf das Polyolefin, eingesetzt. Das organische Phosphit oder Phosphonit wird zweckmässig in einer Menge von 0,01 bis 2, insbesondere 0,01 bis 1 Gew. %, ebenfalls bezogen auf das Polyolefin, eingesetzt. Als organische Phosphite bzw. Phosphonite werden vorzugsweise solche eingesetzt, wie sie in der deutschen Offenlegungsschrift DE-A- 42 02 276 beschrieben sind. Siehe dort insbesondere die Patentansprüche, die Beispiele sowie die Seiten 4, letzter Absatz bis Seite 8. Besonders zweckmässige Phosphite und Phosphonite sind auch Punkt 4 der obigen Auflistung von Costabilisatoren zu entnehmen.

Eine speziell ausgezeichnete Stabilisierung von Polyolefinen wird beispielsweise dann erhalten, wenn die erfindungsgemässen Verbindungen in einer Dreierkombination mit organischen Phosphiten oder Phosphoniten und einem phenolischen Antioxidans eingesetzt werden.

Ebenfalls Gegenstand der Erfindung sind neue Verbindungen der Formel Ia,

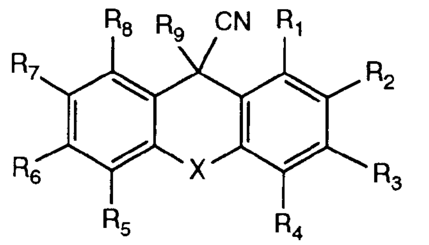

(Ia)

worin $R_1$, $R_2$, $R_3$, $R_4$, $R_5$, $R_6$, $R_7$ und $R_6$ unabhängig voneinander Wasserstoff, $C_1$-$C_{25}$-Alkyl, durch Sauerstoff, Schwefel oder $>$N-$R_{10}$ unterbrochenes $C_2$-$C_{25}$-Alkyl; unsubstituiertes oder durch $C_1$-$C_4$-Alkyl substituiertes $C_5$-$C_8$-Cycloalkyl; unsubstituiertes oder durch $C_1$-$C_4$-Alkyl substituiertes Phenyl; $C_7$-$C_9$-Phenylalkyl, Hydroxy, $C_1$-$C_{18}$-Alkoxy, durch Sauerstoff, Schwefel oder $>$N-$R_{10}$ unterbrochenes $C_3$-$C_{18}$-Alkoxy; Mercapto, $C_1$-$C_{18}$-Alkylthio, durch Sauerstoff, Schwefel oder $>$N-$R_{10}$ unterbrochenes $C_3$-$C_{18}$-Alkylthio; $C_1$-$C_{25}$-Alkanoyloxy, durch Sauerstoff, Schwefel oder $>$N-$R_{10}$ unterbrochenes $C_3$-$C_{25}$-Alkanoyloxy; $C_3$-$C_{25}$-Alkenoyloxy, Benzoyloxy oder durch $C_1$-$C_{12}$-Alkyl substituiertes Benzoyloxy; Nitro, Cyano, oder $-(CH_2)_mCOR_{11}$ bedeuten, ferner die Reste $R_1$ und $R_2$, $R_2$ und $R_3$, $R_3$ und $R_4$, $R_6$ und $R_6$, $R_6$ und $R_7$ oder $R_7$ und $R_8$ zusammen mit den Kohlenstoffatomen, an die sie gebunden sind, einen Benzoring bilden, oder die gleichen Rest-Paare zusammen $-O(CH_2)_nO-$ bedeuten, mit der Bedingung, dass mindestens einer der Reste $R_1$, $R_2$, $R_3$ oder $R_4$, sowie mindestens einer der Reste $R_6$, $R_6$, $R_7$ oder $R_8$ Wasserstoff darstellen, mindestens einer der Reste $R_1$, $R_2$, $R_3$, $R_4$, $R_5$, $R_6$, $R_7$ und $R_6$ von Wasserstoff verschieden ist, und wenn $R_1$, $R_2$ oder $R_3$ Methyl oder Methoxy bedeutet, mindestens einer der Reste $R_4$, $R_6$, $R_6$, $R_7$ oder $R_6$ von Wasserstoff verschieden ist, $R_1$ zusätzlich einen Rest der Formel IIa, $R_2$ zusätzlich einen Rest der Formel IIIa, $R_3$ zusätzlich einen Rest der Formel IVa und $R_4$ zusätzlich einen Rest der Formel Va bedeutet, wobei gleichzeitig nur ein Rest der Formel IIa, IIIa, IVa oder Va in der Verbindung der Formel Ia auftritt,

(IIa)

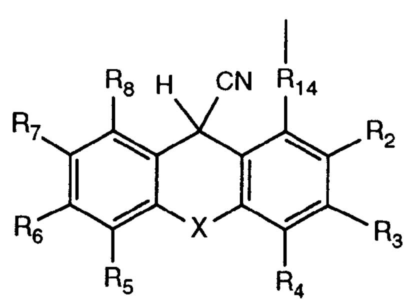

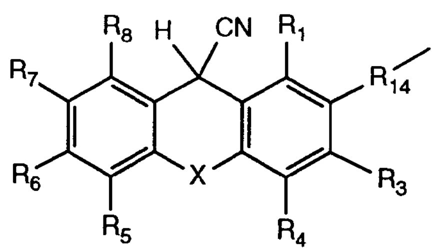

(IIIa)

(IVa) 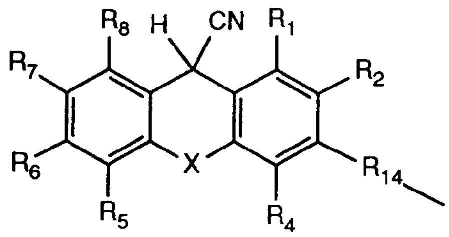 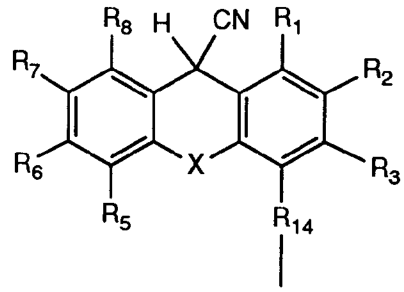 (Va)

$R_9$ Wasserstoff oder einen Rest der Formel VIa

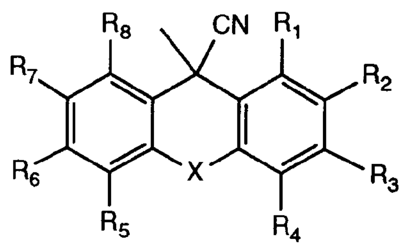 (VIa)

darstellt, wobei $R_1$, $R_2$, $R_3$ und $R_4$ nicht einen Rest der Formel IIa, IIIa, IVa oder Va bedeuten,
$R_{10}$ Wasserstoff oder $C_1$-$C_8$-Alkyl darstellt,
$R_{11}$ Hydroxy, $C_1$-$C_{18}$-Alkoxy oder

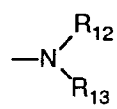

bedeutet,
$R_{12}$ und $R_{13}$ unabhängig voneinander Wasserstoff oder $C_1$-$C_{18}$-Alkyl darstellen,
$R_{14}$ eine direkte Bindung, $C_1$-$C_{18}$-Alkylen, durch Sauerstoff, Schwefel oder >N-$R_{10}$ unterbrochenes $C_2$-$C_{18}$-Alkylen;

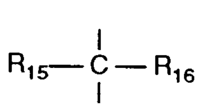 oder 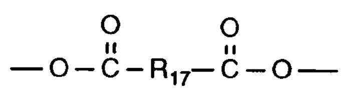

bedeutet,
$R_{15}$ und $R_{16}$ unabhängig voneinander Wasserstoff, $CF_3$, $C_1$-$C_{12}$-Alkyl oder Phenyl darstellen, oder $R_{15}$ und $R_{16}$ zusammen mit dem C-Atom, an das sie gebunden sind, einen unsubstituierten oder durch 1 bis 3 $C_1$-$C_4$-Alkyl substituierten $C_5$-$C_8$-Cycloalkylidenring bilden,
$R_{17}$ eine direkte Bindung, $C_1$-$C_{18}$-Alkylen, durch Sauerstoff, Schwefel oder >N-$R_{10}$ unterbrochenes $C_2$-$C_{18}$-Alkylen; $C_2$-$C_{18}$-Alkenylen, $C_2$-$C_{20}$-Alkyliden, $C_7$-$C_{20}$-Phenylalkyliden, $C_5$-$C_8$-Cycloalkylen, $C_7$-$C_8$-Bicycloalkylen, unsubstituiertes oder durch $C_1$-$C_4$-Alkyl substituiertes Phenylen,

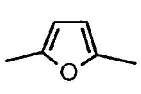 oder 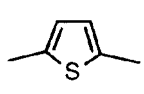

darstellt,
$R_{18}$ Wasserstoff, $C_1$-$C_{25}$-Alkyl, durch Sauerstoff, Schwefel oder >N-$R_{10}$ unterbrochenes $C_2$-$C_{25}$-Alkyl; unsubstituiertes oder durch $C_1$-$C_4$-Alkyl substituiertes $C_5$-$C_8$-Cycloalkyl; unsubstituiertes oder durch $C_1$-$C_4$-Alkyl substituiertes Phenyl; $C_7$-$C_9$-Phenylalkyl, Hydroxy, $C_1$-$C_{18}$-Alkoxy, durch Sauerstoff, Schwefel oder >N-$R_{10}$ un-

terbrochenes $C_3$-$C_{18}$-Alkoxy; $C_1$-$C_{25}$-Alkanoyl, durch Sauerstoff, Schwefel oder >N-$R_{10}$ unterbrochenes $C_3$-$C_{25}$-Alkanoyl; $C_3$-$C_{25}$-Alkenoyl, Benzoyl oder durch $C_1$-$C_{12}$-Alkyl substituiertes Benzoyl; Cyano, $-(CH_2)_mCOR_{11}$ oder

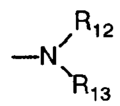

bedeutet,
$R_{19}$ $C_1$-$C_{25}$-Alkyl, $C_7$-$C_9$-Phenylalkyl oder $-CH_2CH_2OH$ darstellt,
X eine direkte Bindung, Sauerstoff, Schwefel, $-SO-$, $-SO_2-$, $-CH_2-$, $-CH_2CH_2-$, $-CH=CH-$,

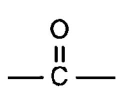 , 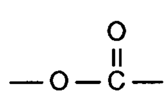 , 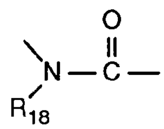 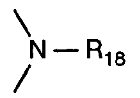 , 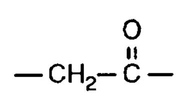

oder

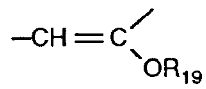

bedeutet,
m 0, 1 oder 2, und
n 1 oder 2 darstellt.

Bevorzugte Gruppen von neuen Verbindungen der Formel Ia entsprechen den in den oben für die erfindungsgemässen Zusammensetzungen ausgedrückten Bevorzugungen.

Bevorzugt sind ausserdem Verbindungen der Formel Ia, worin $R_5$, $R_6$, $R_7$ und $R_8$ Wasserstoff bedeuten, mit der Bedingung, dass mindestens einer der Reste $R_1$, $R_2$, $R_3$ oder $R_4$ von Wasserstoff verschieden ist, und keiner der Reste $R_1$, $R_2$ und $R_3$ Methyl oder Methoxy bedeutet.

Besonders bevorzugt sind Verbindungen der Formel Ia, worin
$R_1$, $R_2$, $R_3$ und $R_4$ unabhängig voneinander Wasserstoff, $C_4$-$C_{18}$-Alkyl, $C_5$-$C_8$-Cycloalkyl, Phenyl, $C_7$-$C_9$-Phenylalkyl, Hydroxy, $C_3$-$C_{18}$-Alkoxy, $C_1$-$C_{18}$-Alkanoyloxy, $C_3$-$C_{18}$-Alkenoyloxy, Benzoyloxy, Cyano oder $-(CH_2)_mCOR_{11}$ bedeuten, mit der Bedingung, dass mindestens einer der Reste $R_1$, $R_2$, $R_3$ oder $R_4$ Wasserstoff darstellt, $R_2$ zusätzlich einen Rest der Formel IIIa und $R_4$ zusätzlich einen Rest der Formel Va bedeutet, wobei gleichzeitig nur ein Rest der Formel IIIa oder Va in der Verbindung der Formel Ia auftritt,

(IIIa) 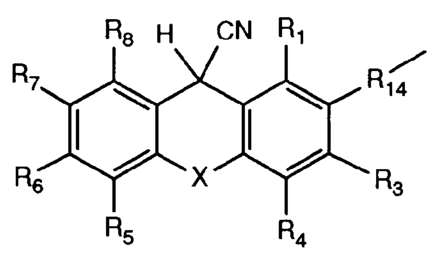 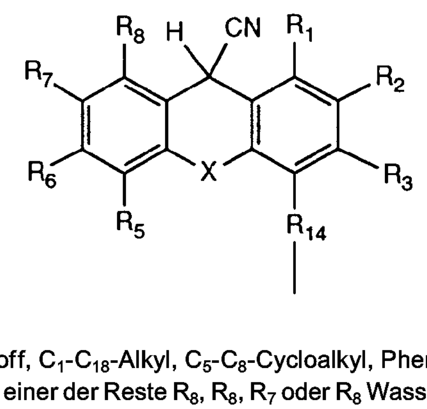 (Va)

$R_5$, $R_8$, $R_7$ und $R_8$ unabhängig voneinander Wasserstoff, $C_1$-$C_{18}$-Alkyl, $C_5$-$C_8$-Cycloalkyl, Phenyl, Benzyl oder Cyano bedeuten, mit der Bedingung, dass mindestens einer der Reste $R_8$, $R_8$, $R_7$ oder $R_8$ Wasserstoff darstellt, und mindestens einer der Reste $R_1$, $R_2$, $R_3$, $R_4$, $R_5$, $R_6$, $R_7$ und $R_8$ von Wasserstoff verschieden ist,
$R_{11}$ $C_1$-$C_{12}$-Alkoxy ist,
$R_{14}$ $C_1$-$C_8$-Alkylen, durch Sauerstoff unterbrochenes $C_2$-$C_{12}$-Alkylen oder

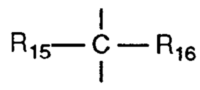

bedeutet,
$R_{15}$ und $R_{16}$ unabhängig voneinander Wasserstoff, $C_1$-$C_8$-Alkyl oder Phenyl darstellen,
oder $R_{15}$ und $R_{16}$ zusammen mit dem C-Atom, an das sie gebunden sind, einen $C_5$-$C_7$-Cycloalkylidenring bilden,
$R_{18}$ Wasserstoff, $C_1$-$C_{12}$-Alkyl, Benzyl, Hydroxy, $C_1$-$C_{12}$-Alkoxy, $C_1$-$C_{18}$-Alkanoyl oder Benzoyl bedeutet,
X eine direkte Bindung, Sauerstoff, Schwefel, $-CH_2CH_2-$, -CH=CH- oder >N-$R_{18}$ darstellt, und
m 0 oder 1 ist.

Die Verbindungen der Formel I und Ia können auf an sich bekannte Weise hergestellt werden.

Beispielsweise wird eine Carbonsäure der Formel VII,

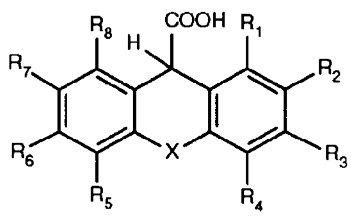

(VII)

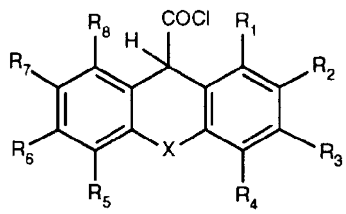

(VIII)

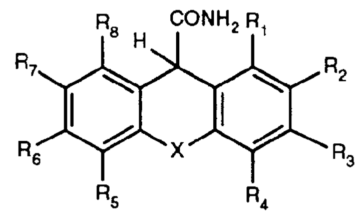

(IX)

worin $R_1$, $R_2$, $R_3$, $R_4$, $R_5$, $R_6$, $R_7$ und $R_6$ die angegebenen Bedeutungen haben und X bevorzugt eine direkte Bindung bedeutet, in Analogie zu US-A-3 452 076 und in Analogie zu Vorschriften von M.N. Romanelli et al, Il Farmaco 46(10), 1121 (1991) via Säurechlorid der Formel VIII und Amid der Formel IX zum Nitril der Formel I umgesetzt.

Wenn X beispielsweise Sauerstoff oder Schwefel bedeutet, werden die Xanthydrole beziehungsweise Thioxanthydrole der Formel XI in Analogie zu US-A-2 956 063 mit beispielsweise Kaliumcyanid in Essigsäure zu den Verbindungen der Formel I umgesetzt.

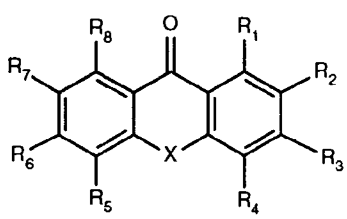

(X)

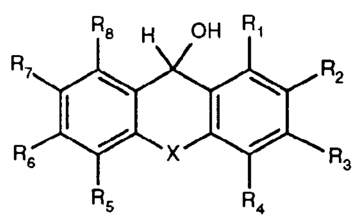

(XI)

Die Verbindungen der Formel XI, worin X Sauerstoff, Schwefel, $-CH_2CH_2-$, -CH=CH- oder >N-$R_{18}$ bedeutet, können auch beispielsweise mit Trimethylsilylcyanid und einer katalytischen Menge Zinkiodid in Dichlormethan in Analogie zu Vorschriften von K. Kobayashi et al., J. Chem. Soc. Chem. Commun. 1992, 780, zu den Verbindungen der Formel I bzw. Ia umgesetzt werden.

Die Xanthydrole beziehungsweise Thioxanthydrole der Formel XI sind in der Literatur bekannt und werden bevorzugt aus den bekannten Xanthonen bzw. Thioxanthonen der Formel X (X = römische Zahl zehn), worin X (X = Buchstabe) Sauerstoff oder Schwefel bedeutet, durch Reduktion der Carbonylgruppe hergestellt.

Die Herstellung von Xanthonen bzw. Thioxanthonen der Formel X erfolgt bevorzugt beispielsweise durch Ringschluss unter Wasserabspaltung von o-Phenoxybenzoesäure- oder o-Phenylthiobenzoesäure-Derivaten.

Eine weitere Möglichkeit zur Herstellung der Verbindungen der Formel I und Ia betrifft die Umsetzung von bekannten Xanthylium- (X = Sauerstoff), Thioxanthylium- (X = Schwefel) oder Acridinium (X = >N-$R_{10}$ )-Salzen der Formel XII,

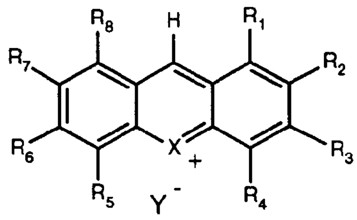

(XII)

worin Y ein Anion, wie beispielsweise Halogenid, Perchlorat oder Hydrogensulfat bedeutet, in Analogie zu einer Vorschrift von M. Hori et al, J. Org. Chem. 45 (12), 2468 (1980) mit Cyanid, wie beispielsweise Kaliumcyanid, in einem wässrigen oder organischen Lösungsmittel.

Die Reaktionstemperaturen liegen zwischen Raumtemperatur und 150°C, bevorzugt zwischen 20 und 80°C.

Die Verbindungen der Formel I und Ia, worin X $-CH_2CH_2-$ oder -CH=CH- bedeutet, werden in Analogie zu V. Valenta et al, Coll. Czech. Chem. Comm. 53 (4), 860 (1988) oder M.A. Davis et al, J. Med. Chem. 7, 88 (1964) ausgehend von bekannten Halogeniden der Formel XIII

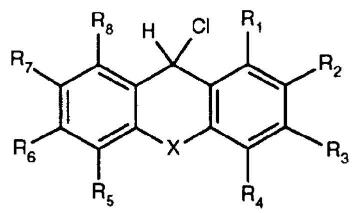

(XIII)

durch Umsetzung mit Cyaniden, wie beispielsweise Trimethylsilylcyanid oder Silbercyanid, und einer katalytischen Menge einer Lewis-Säure, wie beispielsweise Zinntetrachlorid, in einem organischen Lösungsmittel, wie beispielsweise Methylenchlorid oder Toluol, hergestellt.

Die Verbindungen der Formel XIII, worin X $-CH_2CH_2-$ oder -CH=CH- bedeutet, sind in der Literatur bekannt oder können beispielsweise gemäss V. Mychajlyszyn et al, Coll. Czech. Chem. Comm. 24, 3955 (1959); oder G. Berti, Gazz. Chim. Ital. 87, 293 (1957) in analoger Weise hergestellt werden.

Die Verbindungen der Formel I und Ia, worin X

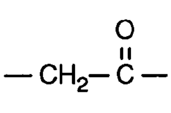 oder 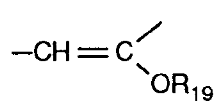

bedeutet, werden in Analogie zu M.A. Davis et al, Can. J. Chem. 47 (15), 2827 (1969) oder US-A-3 641 038 hergestellt.

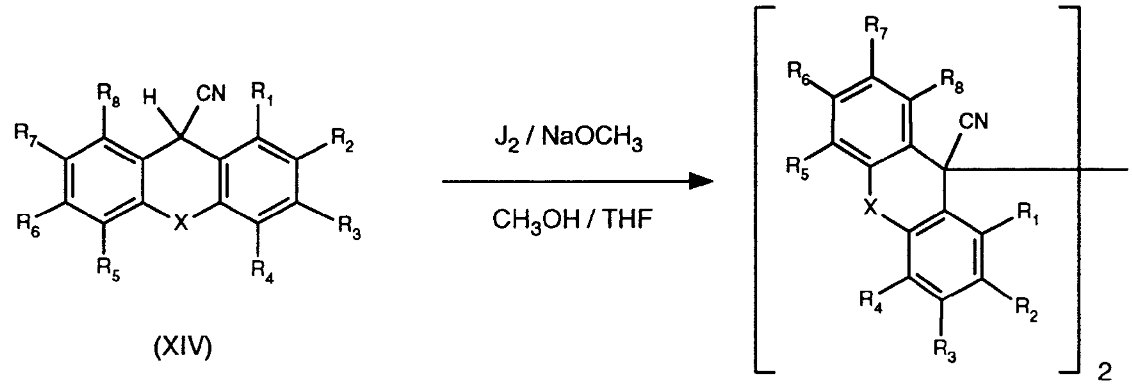


(XIV)

(XV)

Die Dimerisierung der Verbindungen der Formel XIV, zur Herstellung von Verbindungen der Formel I und Ia, worin $R_9$ einen Rest der Formel VI oder VIa darstellt [Verbindungen der Formel XV] erfolgt durch Oxidation mit beispielsweise Jod unter basischen Bedingungen in einem organischen Lösungsmittel bei Raumtemperatur. Als Base eignet sich besonders Natriummethylat, als Lösungsmittel Methanol, Diethylether oder Tetrahydrofuran.

Die folgenden Beispiele erläutern die Erfindung weiter. Angaben in Teilen oder Prozenten beziehen sich auf das Gewicht.

Beispiel 1: Herstellung von 9-Cyano-fluoren (Verbindung (101), Tabelle 1).

Das 9-Cyano-fluoren ist in US-A-3 452 076 beschrieben oder kann in Analogie zu M.N. Romanelli et al, Il Farmaco 46(10), 1121 (1991) hergestellt werden.

21,0 g (0,10 Mol) Fluoren-9-carbonsäure werden mit 100 ml (1,37 Mol) Thionylchlorid versetzt und während ca. 90 Minuten unter Rückfluss gekocht. Der Ueberschuss Thionylchlorid wird am Vakuumrotationsverdampfer eingeengt. Der Rückstand wird dreimal mit je ca. 50 ml Toluol aufgenommen und am Vakuumrotationsverdampfer eingeengt. Der ölige Rückstand wird portionenweise zu einer Mischung von ca. 1 kg Eis und 240 ml konzentrierter Ammoniak-Lösung gegeben. Das ausgefallene Fluoren-9-carbonsäureamid wird abfiltriert, mit Wasser gewaschen und am Hochvakuum getrocknet. Anschliessend wird das getrocknete Fluoren-9-carbonsäureamid zu 160 ml (1,75 Mol) Phosphoroxychlorid gegeben und während ca. 2 Stunden unter Rückfluss gekocht. Das Reaktionsgemisch wird abgekühlt und am Vakuumrotationsverdampfer eingeengt. Der Rückstand wird in Toluol aufgenommen und mit Wasser gewaschen. Die organischen Phasen werden vereinigt, über Natriumsulfat getrocknet und am Vakuumrotationsverdampfer eingeengt. Kristallisation der Rückstandes aus Cyclohexan liefert 12,4 g (65 %) 9-Cyano-fluoren, Smp. 152-154°C (Verbindung (101), Tabelle 1).

Beispiel 2: Herstellung von 9-Cyano-xanthen (Verbindung (102), Tabelle 1).

Die Herstellung von 9-Cyano-xanthen ist beispielsweise in US-A-2 956 063 beschrieben.

Zu einer auf 50°C erwärmten Lösung von 24,2 g (0,122 Mol) Xanthydrol in 420 ml Essigsäure werden portionenweise 17,5 g (0,268 Mol) Kaliumcyanid gegeben. Anschliessend wird das Reaktionsgemisch während 2 Stunden bei 60°C gerührt, dann abgekühlt, auf 400 ml Wasser gegossen und mit Essigester/Hexan = 1:1 extrahiert. Die organischen Phasen werden mit Wasser gewaschen, vereinigt, über Natriumsulfat getrocknet und am Vakuumrotationsverdampfer eingeengt. Chromatographie des Rückstandes an Kieselgel mit dem Laufmittelsystem Hexan/Essigester = 19:1 und Kristallistion der reinen Fraktionen aus Ether/Petrolether liefert 20,2 g (80 %) 9-Cyano-xanthen, Smp. 101°C (Verbindung (102), Tabelle 1).

In Analogie zu Beispiel 2 werden aus den entsprechenden substituierten Xanthydrolen und Thioxanthydrolen, wie beispielsweise 4-Cyclohexyl-xanthydrol, 2-tert-Butyl-xanthydrol, 2-Nonyl-xanthydrol, 2-(α,α-Dimethylbenzyl)-4-methyl-xanthydrol, 2-n-Propoxy-xanthydrol, 2,4-Diethylthioxanthydrol oder 2-Dodecyl-thioxanthydrol die Verbindungen (111), (112), (113), (114), (115), (116) und (117) hergestellt.

a) Herstellung der substituierten Xanthydrole und Thioxanthydrole:

Zu einer Suspension von 1,4 g (36,0 mMol) Lithiumaluminiumhydrid in 10 ml absolutem Tetrahydrofuran wird eine Lösung von 13,9 g (50,0 mMol) 4-Cyclohexyl-xanthon in 150 ml absolutem Tetrahydrofuran getropft. Das Reaktionsgemisch wird während 1 Stunde bei Raumtemperatur gerührt, dann auf 10°C ab-

gekühlt, mit 100 ml Diethylether verdünnt und nacheinander mit 1,5 ml Wasser, 1,5 ml einer 15 % wässrigen Natriumhydroxid-Lösung und 4,5 ml Wasser versetzt. Der Niederschlag wird filtriert und das Filtrat am Vakuumrotationsverdampfer eingeengt. Es resultieren 14,0 g (100 %) 4-Cyclohexyl-xanthydrol als braunes Oel, welches ohne weitere Reinigung zum 4-Cyclohexyl-9-cyano-xanthen (Verbindung (111), Tabelle 1) umgesetzt werden kann.

In Analogie zu Beispiel 2a werden aus den entsprechenden substituierten Xanthonen und Thioxanthonen, wie beispielsweise 2-tert-Butyl-xanthon, 2-Nonyl-xanthon, 2-(α,α-Dimethylbenzyl)-4-methyl-xanthon, 2-n-Propoxy-xanthon, 2,4-Diethyl-thioxanthon (Kayacure®DETX) oder 2-Dodecyl-thioxanthon (Ultracure®DTX) das 2-tert-Butyl-xanthydrol, 2-Nonyl-xanthydrol, 2-(α,α-Dimethylbenzyl)-4-methyl-xanthydrol, 2-n-Propoxy-xanthydrol, 2,4-Diethyl-thioxanthydrol oder 2-Dodecyl-thioxanthydrol hergestellt.

b) Herstellung der substituierten Xanthone:

Ein Gemisch von 18,4 g (65 mMol) 2-(2-Cyclohexylphenoxy)benzoesäure und 190 g Polyphosphorsäure wird während 2 Stunden bei 100°C gehalten. Das Reaktionsgemisch wird anschliessend mit 200 ml Methanol verdünnt, mit Natriumcarbonat neutralisiert und auf ca. 800 ml Wasser gegossen. Das ausgefallene Produkt wird filtriert, mit Wasser gewaschen und am Hochvakuum getrocknet. Es resultieren 14,6 g (77 %) 4-Cyclohexylxanthon, Smp. 122-124°C, als beiges Pulver.

In Analogie zu Beispiel 2b werden aus den entsprechenden substituierten Benzoesäuren, wie beispielsweise 2-(4-tert-Butylphenoxy)benzoesäure, 2-(4-Nonylphenoxy)benzoesäure, 2-[4-(α,α-Dimethylbenzyl)-2'-methyl-phenoxy]benzoesäure oder 2-(4-n-Propoxyphenoxy)benzoesäure, das 2-tert-Butyl-xanthon, Smp. 101-103°C; 2-Nonyl-xanthon, Oel (Isomerengemisch); 2-(α,α-Dimethylbenzyl)-4-methyl-xanthon, Smp. 132-134°C; oder 2-n-Propoxy-xanthon, Smp. 94-100°C; hergestellt.

c) Herstellung der substituierten Benzoesäuren:

Eine Mischung von 21,4 g (0,12 Mol) 2-Chlorbenzoesäure-Natriumsalz, 26,1 g (0,132 Mol) Natrium-2-cyclohexylphenolat, 4,95 g (0,05 Mol) Kupfer(I)chlorid und 16,17 g (0,05 Mol) Tris-[2-(2-methoxyethoxy)-ethyl]amin (TDA) in 1,5 l Diethylenglykol-dimethylether ("Diglyme") wird während 8 Stunden bei 140°C gehalten. Anschliessend wird das Lösungsmittel aum Vakuumrotationsverdampfer eingeengt, der Rückstand mit Wasser verdünnt und mit konzentrierter Salzsäure angesäuert. Das Produkt wird mit Essigester extrahiert. Die organischen Phasen werden mit Wasser gewaschen, vereinigt, über Natriumsulfat getrocknet und am Vakuumrotationsverdampfer eingeengt. Es resultieren 22,2 g (68 %) 2-(2-Cyclohexylphenoxy)benzoesäure, Smp. 109-123°C, als beiges Pulver.

In Analogie zu Beispiel 2c werden aus den entsprechenden substituierten Natriumphenolaten, wie beispielsweise Natrium-4-tert-butylphenolat, Natrium-4-nonylphenolat, Natrium-4-(α,α-dimethylbenzyl)-2-methyl-phenolat oder Natrium-4-n-Propoxyphenolat, die 2-(4-tert-Butylphenoxy)benzoesäure, Smp. 137-139°C; 2-(4-Nonylphenoxy)benzoesäure, Oel (Isomerengemisch); 2-[4-(α,α-Dimethylbenzyl)-2-methyl-phenoxy]benzoesäure, Smp. 154-158°C; oder 2-(4-n-Propoxyphenoxy)benzoesäure, Smp. 139-144°C; hergestellt.

Beispiel 3: Herstellung von 9-Cyano-thioxanthen (Verbindung (103), Tabelle 1).

Die Herstellung von 9-Cyano-thioxanthen wurde beispielsweise von M. Hori et al, J. Org. Chem. 45 (12), 2468 (1980) beschrieben.

Unter Stickstoff-Atmosphäre werden zu einem gut gerührten Gemisch von 3,5 g (53,8 mMol) Kaliumcyanid in 4 ml Wasser und 30 ml Methylenchlorid 8,0 g (27,0 mMol) Thioxanthylium-perchlorat [C.C. Price et al, J. Amer. Chem. Soc. 85, 2278 (1963)] gegeben. Nach einer Stunde wird über Kaliumcarbonat getrocknet, filtriert und das Filtrat am Vakuumrotationsverdampfer eingeengt. Kristallisation des Rückstandes aus Isopropanol liefert 5,5 g (91 %) 9-Cyano-thioxanthen, Smp. 103-105°C, farblose Nadeln (Verbindung (103), Tabelle 1).

Beispiel 4: Herstellung von 5-Cyano-10,11-dihydro-5H-dibenzo[a,d]cyclohepten (Verbindung (104), Tabelle 1).

Die Herstellung von 5-Cyano-10,11-dihydro-5H-dibenzo[a,d]cyclohepten wurde beispielsweise von V. Valenta et al, Coll. Czech. Chem. Comm. 53 (4), 860 (1988) beschrieben.

Zu einer Lösung von 21,1 g (92,3 mMol) 5-Chlor-10,11-dihydro-5H-dibenzo[a,d]cyclohepten [V. Mychajlyszyn et al, Coll. Czech. Chem. Comm. 24, 3955 (1959)] und 11,3 g (114 mMol) Trimethylsilylcyanid in 150 ml Methylenchlorid werden langsam 6,0 g (23,0 mMol) Zinntetrachlorid gegeben. Das Reaktionsgemisch wird 10 Stunden bei Raumtemperatur gerührt, dann mit 150 ml Methylenchlorid verdünnt und mit 200 ml Wasser versetzt. Die organische Phase wird abgetrennt, mit verdünnter Natriumbicarbonat-Lösung gewaschen, über Natriumsulfat getrocknet und an Vakuumrotationsverdampfer eingeengt. Kristallisation des Rückstandes aus Toluol liefert 18,0 g (88,9 %) 5-Cyano-10,11-dihydro-5H-dibenzo[a,d]cyclohepten, Smp. 89-90°C (Verbindung (104), Tabelle 1).

Beispiel 5: Herstellung von 5-Cyano-5H-dibenzo[a,d]cyclohepten (Verbindung (105), Tabelle 1).

Die Herstellung von 5-Cyano-5H-dibenzo[a,d]cyclohepten wurde beispielsweise von M.A. Davis et al, J. Med. Chem. 7, 88 (1964) beschrieben.

Zu einer bei 35°C gerührten Suspension von 32,4 g (0,24 Mol) Silbercyanid in 250 ml absolutem Toluol wird während 3 Stunden eine Lösung von 27,4 g (0,12 Mol) 5-Chlor-5H-dibenzo[a,d]cyclohepten [G. Berti, Gazz. Chim. Ital. 87, 293 (1957)] in 250 ml absolutem Toluol getropft. Anschliessend wird noch während 7 Stunden unter Rückfluss gekocht. Das Reaktionsgemisch wird auf Raumtemperatur abgekühlt, filtriert und das Filtrat am Vakuumrotationsverdampfer eingeengt. Kristallisation des Rückstandes aus Hexan liefert 20,0 g (76,7 %) 5-Cyano-5H-dibenzo[a,d]cyclohepten, Smp. 102-104°C (Verbindung (105), Tabelle 1).

Beispiel 6: Herstellung von 9-Cyano-9,10-dihydro-10-methyl-acridin (Verbindung (106), Tabelle 1).

Die Herstellung von 9-Cyano-9,10-dihydro-10-methyl-acridin wurde beispielsweise von A. Kaufmann et al, Ber. Dt. Chem. Ges. 42, 1999 (1909) beschrieben.

Eine Lösung von 10,0 g (43,5 mMol) Acridinchlormethylat [A. Kaufmann et al, Ber. Dt. Chem. Ges. 42, 1999 (1909)] in 100 ml Wasser wird mit 200 ml Diethylether überschichtet und mit einer Lösung von 3,13 g (48,0 mMol) Kaliumcyanid in 20 ml Wasser versetzt. Nach ca. 30 Minuten wird die Etherphase abgetrennt, auf 50 ml am Vakuumrotationsverdampfer eingeengt und das ausgefallene Produkt abfiltriert. Kristallisation des Rohprodukts aus Ethanol liefert 7,10 g (75 %) 9-Cyano-9,10-dihydro-10-methyl-acridin, Smp. 141-143°C (Verbindung (106), Tabelle 1).

Beispiel 7: Herstellung von 9-Cyano-1,3-dihydroxy-xanthen (Verbindung (107), Tabelle 1).

Eine Mischung von 15,5 g (50,0 mMol) 1,3-Dihydroxy-xantheniumbisulfat [R.K.M. Pillai et al, J. Org. Chem. 51, 717 (1986)] und 4,90 g (75,0 mMol) Kaliumcyanid in 150 ml Dimethylformamid wird während 2 Stunden bei 130°C gehalten. Anschliessend wird das Lösungsmittel am Vakuumrotationsverdampfer eingeengt und der Rückstand in Essigester aufgenommen. Die Essigesterphase wird mit Wasser gewaschen, über Natriumsulfat getrocknet und am Vakuumrotationsverdampfer eingeengt. Chromatographie des Rückstandes an Kieselgel mit dem Laufmittelsystem Hexan/Essigester = 3:1 und Kristallisation der reinen Fraktionen aus Essigester liefert 10,0 g (83 %) 9-Cyano-1,3-dihydroxy-xanthen, Smp. 211-215°C (Verbindung (107), Tabelle 1).

Beispiel 8: Herstellung von 9-Cyano-1,3-diacetoxy-xanthen (Verbindung (108), Tabelle 1).

Eine Mischung von 5,50 g (23,0 mMol) 9-Cyano-1,3-dihydroxy-xanthen (Beispiel 7, Verbindung (107), Tabelle 1) und 18,06 g (230 mMol) Acetylchlorid in 55 ml Toluol wird während 10 Stunden bei 80°C gehalten. Anschliessend wird das Reaktionsgemisch am Vakuumrotationsverdampfer leicht eingeengt, der Rückstand mit 10 ml Hexan versetzt und das ausgefallene Produkt filtriert. Es resultieren 6,30 g (85 %) 9-Cyano-1,3-diacetoxy-xanthen, Smp. 160-161°C (Verbindung (108), Tabelle 1).

In Analogie zu Beispiel 8 werden aus den entsprechenden Säurechloriden, wie beispielsweise Pivalinsäurechlorid oder Laurinsäurechlorid die Verbindungen (109) oder (110) hergestellt.

Beispiel 9: Herstellung von 9,9'-Dicyano-9,9'-bis-xanthen (Verbindung (118), Tabelle 1).

Zu einer Lösung von 20,7 g (0,10 Mol) 9-Cyano-xanthen (Beispiel 2, Verbindung (102), Tabelle 1) in 100 ml Tetrahydrofuran werden 19,4 ml (0,105 Mol) einer 5,4 molaren Natriummethylat-Lösung in Methanol getropft. Die gelbe Lösung wird ca. 10 Minuten bei Raumtemperatur gerührt, dann auf +5°C abgekühlt und eine Lösung von 13,0 g (0,051 Mol) Jod in 60 ml Tetrahydrofuran zugetropft. Anschliessend wird das Reaktionsgemisch während 2 Stunden bei Raumtemperatur gerührt. Das Reaktionsgemisch wird am Vakuumrotationsverdampfer leicht eingeengt, mit Wasser versetzt und mit Essigester extrahiert. Die organischen Phasen werden mit Wasser gewaschen, vereinigt, über Natriumsulfat getrocknet und am Vakuumrotationsverdampfer eingeengt. Kristallisation des Rückstandes aus Toluol liefert 7,5 g (36 %) 9,9'-Dicyano-9,9'-bis-xanthen, Smp. 225°C (Verbindung (118), Tabelle 1).

Beispiel 10: Herstellung von 9-Cyano-9H-xanthen-2-carbonsäure-n-dodecylester (Verbindung (119), Tabelle 1).

Zu einer auf 0°C gekühlten Lösung von 3,97 g (40 mMol) Trimethylsilylcyanid und 0,19 g (0,6 mMol) Zinkiodid in 10 ml Dichlormethan wird eine Lösung von 8,21 g (20 mMol) 9-Hydroxy-9H-xanthen-2-carbonsäure-n-dodecylester (Beispiel 10a) in 25 ml Dichlormethan getropft. Das Reaktionsgemisch wird während 30 Minuten bei 0-5°C gerührt und anschliessend auf eine wässrige, gesättigte Natriumhydrogencarbonat-Lösung gegossen. Das Produkt wird mit Dichlormethan extrahiert. Die organischen Phasen werden mit Wasser gewaschen, vereinigt, über Natriumsulfat getrocknet und am Vakuumrotationsverdampfer eingeengt. Das erhaltene feste Rohprodukt wird durch Aufschlämmen in kaltem Ethanol gereinigt. Nach Trocknen am Hochvakuum, werden 6,94 g (83 %) 9-Cyano-9H-xanthen-2-carbonsäure-n-dodecylester, Smp. 65-66,5°C (Verbindung (119), Tabelle 1), als weisses Pulver erhalten.

In Analogie zu Beispiel 10 wird aus 9-Hydroxy-9H-xanthen-2-essigsäureethylester (Beispiel 10b) die Verbindung (120), Tabelle 1, Smp. 76,5-78°C, hergestellt.

a) Herstellung des 9-Hydroxy-9H-xanthen-2-carbonsäure-n-dodecylesters.

Zu einer Lösung von 16,34 g (0,04 Mol) 9-Oxo-9H-xanthen-2-carbonsäure-n-dodecylester (Beispiel 10c) in 150 ml absolutem Tetrahydrofuran werden bei Raumtemperatur portionenweise 2,6 g (0,12 Mol) Lithiumborhydrid zugegeben. Das Reaktionsgemisch wird während 4 Stunden bei Raumtemperatur gerührt und anschliessend vorsichtig auf eine wässrige gesättigte Ammoniumchlorid-Lösung gegossen. Das Produkt wird mit Essigester extrahiert. Die organischen Phasen werden mit einer gesättigten wässrigen Natriumchlorid-Lösung gewaschen, vereinigt, über Natriumsulfat getrocknet und am Vakuumrotationsverdampfer eingeengt. Das erhaltene feste Rohprodukt wird durch Aufschlämmen in Hexan gereinigt. Nach Trocknen am Hochvakuum werden 12,91 g (79 %) 9-Hydroxy-9H-xanthen-2-carbonsäure-n-dodecylester, Smp. 60-62°C, als weisses Pulver erhalten.

b) Herstellung von 9-Hydroxy-9H-xanthen-2-essigsäureethylester.

Zu einer Lösung von 14,2 g (50 mMol) 9-Oxo-9H-xanthen-2-essigsäureethylester (Beispiel 10d) in 130 ml Ethanol werden portionenweise 5,67 g (150 mMol) Natriumborhydrid zugegeben. Die Suspension wird während 15 Stunden bei Raumtemperatur gerührt, anschliessend mit 80 ml Ethanol verdünnt und auf Wasser gegossen. Das Produkt wird mit Essigester extrahiert. Die organischen Phasen werden mit einer wässrigen, gesättigten Natriumchlorid-Lösung gewaschen, vereinigt, über Natriumsulfat getrocknet und am Vakuumrotationsverdampfer eingeengt. Der feste Rückstand wird in Ethanol suspendiert, filtriert und am Hochvakuum getrocknet. Es resultieren 6,1 g (43 %) 9-Hydroxy-9H-xanthen-2-essigsäureethylester, Smp. 214-219°C, als weisses Pulver.

c) Herstellung von 9-Oxo-9H-xanthen-2-carbonsäure-n-dodecylester.

Ein Gemisch von 29,9 g (0,11 Mol) 2-(4-Carboxy-phenoxy)benzoesäure (Beispiel 10e) und 250 ml konzentrierter Schwefelsäure wird während einer Stunde auf 80°C erhitzt. Die erhaltene Lösung wird anschliessend auf ca. 2,5 Liter Wasser gegossen und während 30 Minuten stark gerührt. Das ausgefallene Produkt wird filtriert, mit Wasser gewaschen und am Hochvakuum getrocknet. Es resultieren 23,15 g (85 %) 9-Oxo-9H-xanthen-2-carbonsäure, Smp. > 280°C, als beiges Pulver.

Ein Gemisch von 12,01 g (50 mMol) der obigen 9-Oxo-9H-xanthen-2-carbonsäure, 77,4 g (420 mMol) n-Dodecanol und 0,2 g (2 mMol) konzentrierter Schwefelsäure wird während 6 Stunden bei 140°C gehalten. Die Schwefelsäure wird mit 0,33 g (4 mMol) Natriumacetat neutralisiert und der Ueberschuss an n-Dodecanol destillativ entfernt (98°C / 0,1 mbar). Der braune Rückstand wird in Isopropanol aufgenommen, das ausgefallene Produkt filtriert und am Hochvakuum getrocknet. Es resultieren 16,7 g (82 %) 9-Oxo-9H-xanthen-2-carbonsäure-n-dodecylester, Smp. 79-81°C, als beiges Pulver.

d) Herstellung von 9-Oxo-9H-xanthen-2-essigsäureethylester.

In Analogie zu Beispiel 10c wird aus 2-(4-carboxymethyl-phenoxy)benzoesäure (Beispiel 10f) die 9-Oxo-9H-xanthen-2-essigsäure, Smp. 226-228°C, hergestellt, welche mit Ethanol zum 9-Oxo-9H-xanthen-2-essigsäureethylester, Smp. 97-98°C, umgesetzt wird.

e) Herstellung von 2-(4-Carboxy-phenoxy)benzoesäure.

Eine Mischung von 40 g (0,22 Mol) 4-Hydroxy-benzoesäure-di-natriumsalz, 54 g (0,2 Mol) 2-Iodobenzoesäure-natriumsalz, 1,98 g (0,02 Mol) Kupfer(I)chlorid und 6,47 g (0,02 Mol) Tris-[2-(2-methoxyethoxy)-ethyl]amin (TDA) in 500 ml N-Methylpyrrolidon wird während 4 Stunden bei 180°C gehalten. Anschliessend wird das Lösungsmittel destillativ entfernt, der Rückstand mit ca. 1 Liter Wasser verdünnt und mit konzentrierter Salzsäure angesäuert. Das ausgefallene Produkt wird filtriert und am Hochvakuum getrocknet. Es resultieren 29,7 g (58 %) 2-(4-Carboxy-phenoxy)benzoesäure, Smp. 220-223°C, als braunes Pulver.

f) Herstellung von 2-(4-carboxymethyl-phenoxy)benzoesäure.

In Analogie zu Beispiel 10e wird aus 4-Hydroxyphenylessigsäure-dinatriumsalz mit 2-Iodobenzoesäure-natriumsalz die 2-(4-carboxymethyl-phenoxy)benzoesäure, Smp. 175-179°C, hergestellt.

Tabelle 1:

| Nr. | Verbindung | Smp. (°C) | C(%), H (%), N (%), S (%) (berechnet/gefunden) |
|---|---|---|---|
| 101 | 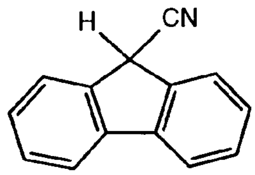 | 152-154 | 87,93 4,74 7,32 —<br>87,70 4,78 7,08 — |
| 102 | 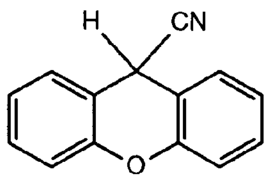 | 101 | 81,14 4,38 6,76 —<br>81,00 4,40 6,60 — |
| 103 | 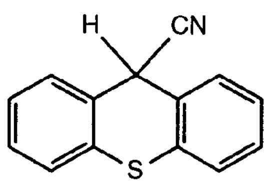 | 103-105 | 75,31 4,06 6,27 14,36<br>75,29 4,07 6,06 14,21 |
| 104 | 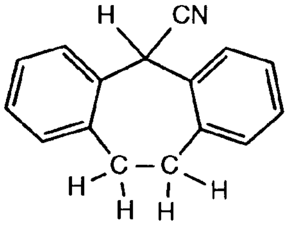 | 89-90 | 87,64 5,98 6,39 —<br>87,49 5,98 6,10 — |

Tabelle 1: (Fortsetzung)

| Nr. | Verbindung | Smp. (°C) | C(%), H (%), N (%), S (%) (berechnet/gefunden) |
|---|---|---|---|
| 105 | 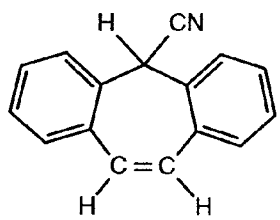 | 102-104 | 88,45 5,10 6,45 —<br>88,35 5,11 6,37 — |
| 106 | 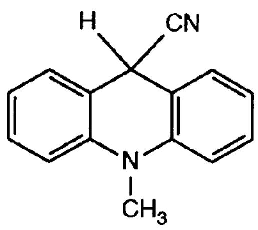 | 141-143 | 81,79 5,49 12,72 —<br>81,70 5,56 12,62 — |
| 107 | 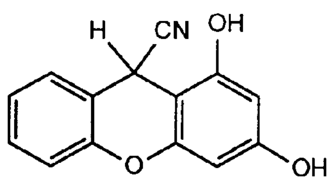 | 211-215 | 70,29 3,79 5,86 —<br>70,22 3,80 6,10 — |
| 108 | 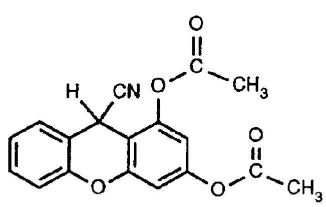 | 160-161 | 66,87 4,05 4,33 —<br>67,04 4,21 4,09 — |

Tabelle 1: (Fortsetzung)

| Nr. | Verbindung | Smp. (°C) | C(%), H (%), N (%), S (%) (berechnet/gefunden) |
|---|---|---|---|
| 109 | 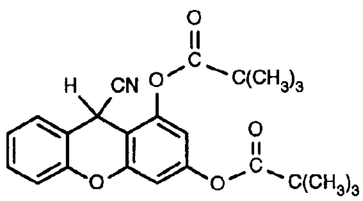 | 148-150 | 70,75 6,18 3,44 —<br>70,78 6,19 3,29 — |
| 110 | 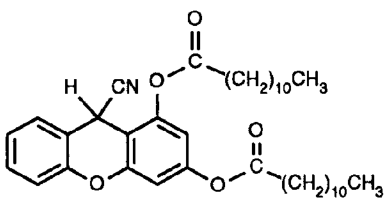 | 67-68 | 75,59 8,85 2,32 —<br>75,69 9,18 2,04 — |
| 111 | 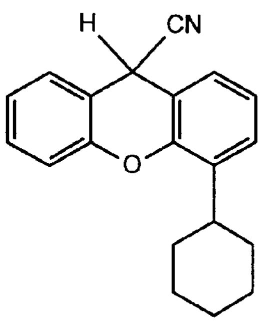 | 97-98 | 83,01 6,62 4,84 —<br>82,98 6,54 4,60 — |
| 112 | 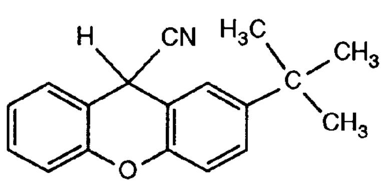 | 67-72 | 82,10 6,51 5,32 —<br>82,12 6,62 4,80 — |

Tabelle 1: (Fortsetzung)

| Nr. | Verbindung | Smp. (°C) | C(%), H (%), N (%), S (%) (berechnet/gefunden) |
|---|---|---|---|
| 113 | 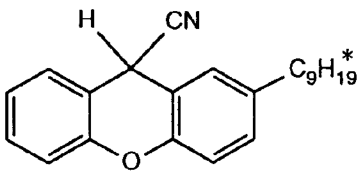 | Oel | 82,84 8,16 4,20 —<br>83,14 8,54 3,52 —<br>* Isomerengemisch |
| 114 | 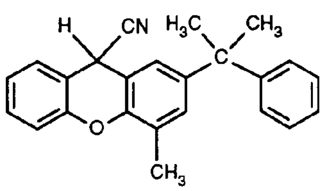 | Oel | 84,92 6,24 4,13 —<br>85,04 6,33 3,99 — |
| 115 | 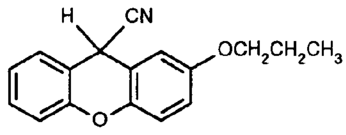 | 80-82 | 76,96 5,70 5,28 —<br>76,86 5,66 5,02 — |
| 116 | 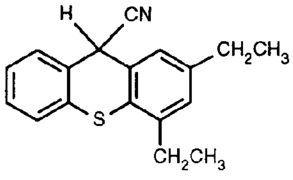 | Oel | 77,38 6,13 5,01 11,47<br>77,45 6,12 4,51 11,49 |

Tabelle 1: (Fortsetzung)

| Nr. | Verbindung | Smp. (°C) | C(%), H (%), N (%), S (%) (berechnet/gefunden) |
|---|---|---|---|
| 117 | 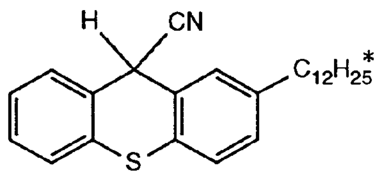 | Oel | 79,74 8,49 3,58 8,19<br>79,74 8,46 3,33 8,52<br>* Isomerengemisch |
| 118 | 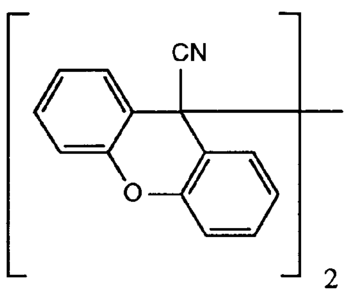 | 225 | 81,54 3,91 6,79 —<br>81,40 3,75 6,62 — |
| 119 | 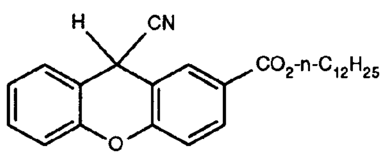 | 65-66,5 | 77,29 7,93 3,34 —<br>77,10 7,82 3,10 — |
| 120 | 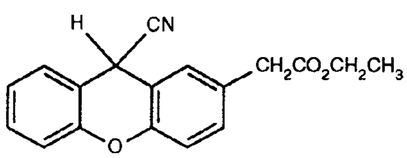 | 76,5-78 | 73,71 5,15 4,78 —<br>73,60 5,28 4,55 — |

Beispiel 11: Stabilisierung von klebrigmachenden Harzen (Tackifiers).

70 g Harz (Bevilite®107, Bergvick) wird mit 0,25 % des zu prüfenden Stabilisators aus Tabelle 1 bei 170°C während 10 Minuten gerührt. Nach dem Formulieren wird das Harz in einem Labormixer pulverisiert. Das Harzpulver wird zu einer Partikelgrösse von 0,1 bis 0,8 mm gesiebt. Ein Teil dieses Harzpulvers wird in offenen Petrischalen bei 40°C in einem Umluftofen während 30 Tagen gealtert und anschliessend der Hydroperoxidgehalt gemessen. Geringere Mengen Peroxide bedeuten bessere Stabilisierung. Der andere Teil wird in einem mit Alufolie abgedeckten "Twist-off Glas" bei 170°C in einem Umluftofen während 48 Stunden gealtert und anschliessend die Gardner Farbe bestimmt. Kleinere Werte bedeuten bessere Stabilisierung. Die Resultate sind in Tabelle 2 zusammengefasst.

Tabelle 2:

| Verbindung aus Tabelle 1 | Hydroperoxidgehalt mMol/g | Gardner Farbe |
|---|---|---|
| — | 0,176 | 10-11 |
| 102 | 0,019 | 7 |
| 103 | 0,021 | 7 |
| 111 | 0,056 | 7-8 |
| 112 | 0,049 | 7-8 |
| 114 | 0,049 | 7-8 |
| 115 | 0,046 | 7 |
| 118 | 0,072 | 7 |

<u>Beispiel 12</u>: Stabilisierung von thermoplastischen Elastomeren auf Styrolbasis

70 g Styrol-Butadien-Styrol (SBS, ®Finapren 416) wird mit 0,25 % des zu prüfenden Stabilisators aus Tabelle 1 in einem Brabender-Plastographen bei 200°C und 60 Umdrehungen pro Minute während 30 Minuten geknetet. Aus dem Verlauf der Drehmomentskurve wird die Induktionszeit ermittelt, d.h. die Knetzeit in Minuten bis zum Anstieg des Drehmoments um 1 Nm nach dem Drehmomentminimum. Grosse Zunahme der Induktionszeit bedeutet gute Stabilisierung. Die Resultate sind in Tabelle 3 zusammengefasst.

Tabelle 3:

| Verbindung aus Tabelle 1 | Induktionszeit in Minuten |
|---|---|
| — | 5,0 |
| 102 | > 100 |
| 103 | > 100 |
| 111 | > 100 |
| 112 | > 100 |
| 113 | > 100 |
| 114 | > 100 |

<u>Beispiel 13</u>: Stabilisierung von Polybutadien-Kautschuk

70 g Polymer (Buna CB 529 C) wird mit 0,25 % des zu prüfenden Stabilisators aus Tabelle 1 in einem Brabender-Plastographen bei 160°C und 60 Umdrehungen pro Minute während 30 Minuten geknetet. Aus dem Verlauf der Drehmomentskurve wird die Induktionszeit ermittelt, d.h. die Knetzeit in Minuten bis zum Anstieg des Drehmoments um 1 Nm nach dem Drehmomentminimum. Grosse Zunahme der Induktionszeit bedeutet gute Stabilisierung. Die Resultate sind in Tabelle 4 zusammengefasst.

Tabelle 4:

| Verbindung aus Tabelle 1 | Induktionszeit in Minuten |
|---|---|
| — | 4,4 |
| 102 | > 180 |
| 111 | > 180 |
| 112 | > 180 |
| 113 | > 180 |
| 114 | > 180 |

Beispiel 14: Stabilisierung von Polypropylen bei Mehrfachextrusion

1,3 kg Polypropylenpulver (Moplen® FL S20), das mit 0,015 % Irganox® 1076 (3-[3,5-di-tert.-butyl-4-hydroxyphenyl]propionsäure-n-octadecylester) vorstabilisiert wurde (mit einem bei 230°C und mit 2,16 kg gemessenen Schmelzindex von 3,2), werden gemischt mit 0,05 % Irganox® 1010 (Pentaerythrityl-tetrakis-[3-(3,5-di-tert.-butyl-4-hydroxyphenyl)-propionat), 0,05 % Calciumstearat, 0,03 % Dihydrotalcit (DHT 4A®, Kyowa Chemical Industry Co., Ltd., $[Mg_{4.5}Al_2(OH)_{13}CO_3 \cdot 3,5\ H_2O]$) und 0,05 % der Verbindung aus Tabelle 1. Diese Mischung wird in einem Extruder mit einem Zylinderdurchmesser von 20 mm und einer Länge von 400 mm mit 100 Umdrehungen pro Minute extrudiert, wobei die 3 Heizzonen auf die folgenden Temperaturen eingestellt werden: 260°, 270°, 280°C. Das Extrudat wird zur Kühlung durch ein Wasserbad gezogen und anschliessend granuliert. Dieses Granulat wird wiederholt extrudiert. Der Schmelzindex wird während der Verarbeitung "online" gemessen und entspricht einem Wert, der konventionell bei 230°C und mit 2.16 kg/10 Min. gemessen würde. Grosse Zunahme des Schmelzindex bedeutet starken Kettenabbau, also schlechte Stabilisierung. Die Resultate sind in Tabelle 5 zusammengefasst.

Tabelle 5:

| Verbindung aus Tabelle 1 | Schmelzindex nach 3 Extrusionen |
|---|---|
| — | 17.8 |
| 101 | 4,7 |
| 102 | 4,5 |
| 111 | 4,5 |
| 112 | 4,7 |
| 113 | 4,6 |
| 114 | 4,6 |
| 115 | 4,7 |
| 116 | 4,5 |
| 117 | 4,6 |

Beispiel 15: Stabilisierung von Polyethylen während der Verarbeitung

100 Teile Polyethylenpulver (Lupolen® 5260 Z) werden mit 0,1 Teilen Irganox® 1010 (Pentaerythrityl-tetrakis-[3-(3,5-di-tert.-butyl-4-hydroxyphenyl)-propionat] und 0,05 Teilen Stabilisator aus Tabelle 1 gemischt und in einem Brabender Plastographen bei 220°C und 50 Umdrehungen pro Minute geknetet. Während dieser Zeit wird der Knetwiderstand als Drehmoment kontinuierlich registriert. Im Verlauf der Knetzeit beginnt das Polymere nach längerer Konstanz zu vernetzen, was anhand der raschen Zunahme des Drehmoments festgestellt werden kann. In der Tabelle 6 ist die Zeit bis zur merklichen Zunahme des Drehmoments als Mass der

Stabilisatorwirkung angegeben.

Tabelle 6:

| Verbindung aus Tabelle 1 | Zeit bis zum Drehmomentanstieg (Min.) |
|---|---|
| — | 5,0 |
| 111 | 38,0 |
| 112 | 37,5 |
| 114 | 43,0 |
| 115 | 41,0 |

**Patentansprüche**

1. Zusammensetzung enthaltend
   a) ein dem oxidativen, thermischen oder lichtinduzierten Abbau unterworfenes organisches Material und
   b) mindestens eine Verbindung der Formel I,

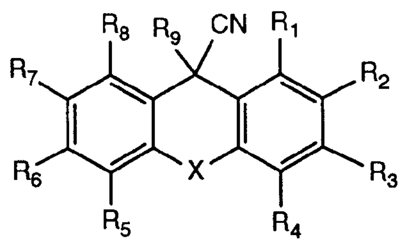

(I)

worin $R_1$, $R_2$, $R_3$, $R_4$, $R_5$, $R_5$, $R_7$ und $R_5$ unabhängig voneinander Wasserstoff, Halogen, $C_1$-$C_{25}$-Alkyl, durch Sauerstoff, Schwefel oder $>$N-$R_{10}$ unterbrochenes $C_2$-$C_{25}$-Alkyl; unsubstituiertes oder durch $C_1$-$C_4$-Alkyl substituiertes $C_5$-$C_8$-Cycloalkyl; unsubstituiertes oder durch $C_1$-$C_4$-Alkyl substituiertes Phenyl; $C_7$-$C_9$-Phenylalkyl, Hydroxy, $C_1$-$C_{18}$-Alkoxy, durch Sauerstoff, Schwefel oder $>$N-$R_{10}$ unterbrochenes $C_3$-$C_{18}$-Alkoxy; Mercapto, $C_1$-$C_{18}$-Alkylthio, durch Sauerstoff, Schwefel oder $>$N-$R_{10}$ unterbrochenes $C_3$-$C_{18}$-Alkylthio; $C_1$-$C_{25}$-Alkanoyloxy, durch Sauerstoff, Schwefel oder $>$N-$R_{10}$ unterbrochenes $C_3$-$C_{25}$-Alkanoyloxy; $C_3$-$C_{25}$-Alkenoyloxy, Benzoyloxy oder durch $C_1$-$C_{12}$-Alkyl substituiertes Benzoyloxy; Nitro, Cyano, -$(CH_2)$m$COR_{11}$ oder

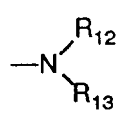

bedeuten, ferner die Reste $R_1$ und $R_2$, $R_2$ und $R_3$, $R_3$ und $R_4$, $R_5$ und $R_6$, $R_5$ und $R_7$ oder $R_7$ und $R_5$ zusammen mit den Kohlenstoffatomen, an die sie gebunden sind, einen Benzoring bilden, oder die gleichen Rest-Paare zusammen -$O(CH_2)_nO$- bedeuten, mit der Bedingung, dass mindestens einer der Reste $R_1$, $R_2$, $R_3$ oder $R_4$, sowie mindestens einer der Reste $R_5$, $R_6$, $R_7$ oder $R_5$ Wasserstoff darstellen, $R_1$ zusätzlich einen Rest der Formel II, $R_2$ zusätzlich einen Rest der Formel III, $R_3$ zusätzlich einen Rest der Formel IV und $R_4$ zusätzlich einen Rest der Formel V bedeutet, wobei gleichzeitig nur ein Rest der Formel II, III, IV oder V in der Verbindung der Formel I auftritt,

(II) 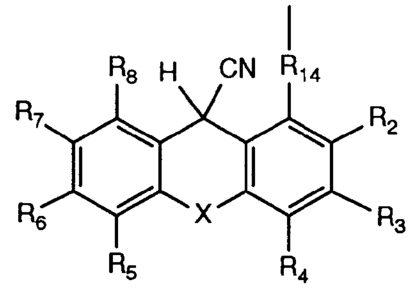 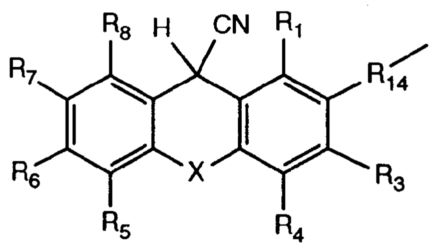 (III)

(IV) 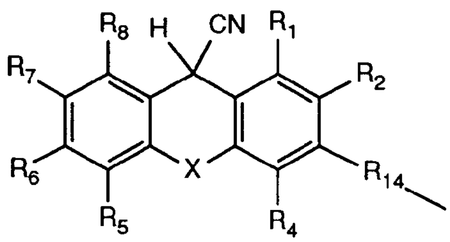 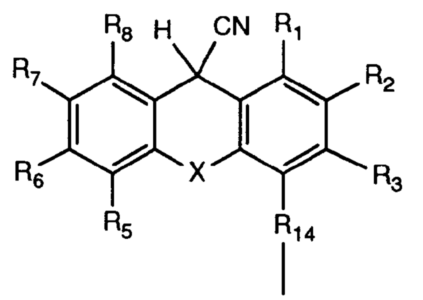 (V)

$R_9$ Wasserstoff oder einen Rest der Formel VI

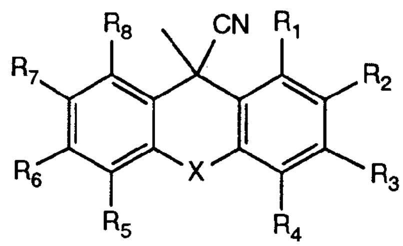 (VI)

darstellt, wobei $R_1$, $R_2$, $R_3$ und $R_4$ nicht einen Rest der Formel II, III, IV oder V bedeuten,
$R_{10}$ Wasserstoff oder $C_1$-$C_8$-Alkyl darstellt,
$R_{11}$ Hydroxy, $C_1$-$C_{18}$-Alkoxy oder

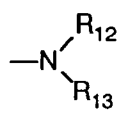

bedeutet,
$R_{12}$ und $R_{13}$ unabhängig voneinander Wasserstoff oder $C_1$-$C_{18}$-Alkyl darstellen,
$R_{14}$ eine direkte Bindung, $C_1$-$C_{18}$-Alkylen, durch Sauerstoff, Schwefel oder $>N-R_{10}$ unterbrochenes $C_2$-$C_{18}$-Alkylen;

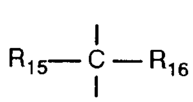 oder 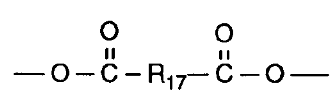

bedeutet,
$R_{15}$ und $R_{16}$ unabhängig voneinander Wasserstoff, $CF_3$, $C_1$-$C_{12}$-Alkyl oder Phenyl darstellen, oder $R_{15}$ und $R_{16}$ zusammen mit dem C-Atom, an das sie gebunden sind, einen unsubstituierten oder durch 1 bis 3 $C_1$-$C_4$-Alkyl substituierten $C_5$-$C_8$-Cycloalkylidenring bilden,
$R_{17}$ eine direkte Bindung, $C_1$-$C_{18}$-Alkylen, durch Sauerstoff, Schwefel oder $>NR_{-10}$ unterbrochenes $C_2$-$C_{18}$-

Alkylen; $C_2$-$C_{18}$-Alkenylen, $C_2$-$C_{20}$-Alkyliden, $C_7$-$C_{20}$-Phenylalkyliden, $C_5$-$C_8$-Cycloalkylen, $C_7$-$C_8$-Bicycloalkylen, unsubstituiertes oder durch $C_1$-$C_4$-Alkyl substituiertes Phenylen,

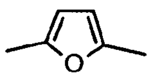 oder 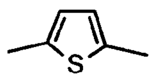

darstellt,
$R_{18}$ Wasserstoff, $C_1$-$C_{25}$-Alkyl, durch Sauerstoff, Schwefel oder $>$N-$R_{10}$ unterbrochenes $C_2$-$C_{25}$-Alkyl; unsubstituiertes oder durch $C_1$-$C_4$-Alkyl substituiertes $C_5$-$C_8$-Cycloalkyl; unsubstituiertes oder durch $C_1$-$C_4$-Alkyl substituiertes Phenyl; $C_7$-$C_9$-Phenylalkyl, Hydroxy, $C_1$-$C_{18}$-Alkoxy, durch Sauerstoff, Schwefel oder $>$N-$R_{10}$ unterbrochenes $C_3$-$C_{18}$-Alkoxy; $C_1$-$C_{25}$-Alkanoyl, durch Sauerstoff, Schwefel oder $>$N-$R_{10}$ unterbrochenes $C_3$-$C_{25}$-Alkanoyl; $C_3$-$C_{25}$-Alkenoyl, Benzoyl oder durch $C_1$-$C_{12}$-Alkyl substituiertes Benzoyl; Cyano, $-(CH_2)_mCOR_{11}$ oder

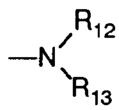

bedeutet,
$R_{19}$ $C_1$-$C_{25}$-Alkyl, $C_7$-$C_9$-Phenylalkyl oder $-CH_2CH_2OH$ darstellt,
X eine direkte Bindung, Sauerstoff, Schwefel, $-SO-$, $-SO_2-$, $-CH_2-$, $-CH_2CH_2-$, $-CH=CH-$,

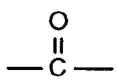 , 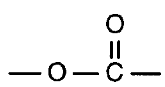 , 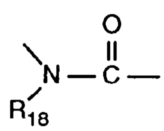 , 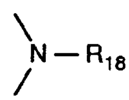 , 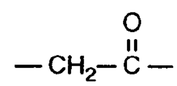

oder

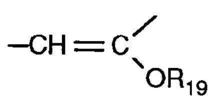

bedeutet,
m 0, 1 oder 2, und
n 1 oder 2 darstellt.

**2.** Zusammensetzung gemäss Anspruch 1, worin
$R_1$, $R_2$, $R_3$, $R_4$, $R_5$, $R_6$, $R_7$ und $R_8$ unabhängig voneinander Wasserstoff, Chlor, $C_1$-$C_{18}$-Alkyl, durch Sauerstoff, Schwefel oder $>$N-$R_{10}$ unterbrochenes $C_2$-$C_{18}$-Alkyl; unsubstituiertes oder durch $C_1$-$C_4$-Alkyl substituiertes $C_5$-$C_8$-Cycloalkyl; unsubstituiertes oder durch $C_1$-$C_4$-Alkyl substituiertes Phenyl; $C_7$-$C_9$-Phenylalkyl, Hydroxy, $C_1$-$C_{18}$-Alkoxy, durch Sauerstoff, Schwefel oder $>$N-$R_{10}$ unterbrochenes $C_3$-$C_{18}$-Alkoxy; Mercapto, $C_1$-$C_{18}$-Alkylthio, $C_1$-$C_{18}$-Alkanoyloxy, durch Sauerstoff, Schwefel oder $>$N-$R_{10}$ unterbrochenes $C_3$-$C_{18}$-Alkanoyloxy; $C_3$-$C_{18}$-Alkenoyloxy, Benzoyloxy oder durch $C_1$-$C_8$-Alkyl substituiertes Benzoyloxy; Nitro, Cyano, $-(CH_2)_mCOR_{11}$ oder

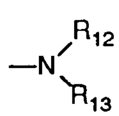

bedeuten, ferner die Reste $R_1$ und $R_2$, $R_2$ und $R_3$, $R_3$ und $R_4$, $R_5$ und $R_6$, $R_8$ und $R_7$ oder $R_7$ und $R_8$ zusammen mit den Kohlenstoffatomen, an die sie gebunden sind, einen Benzoring bilden, oder die gleichen Rest-Paare zusammen $-O(CH_2)_nO-$ bedeuten, mit der Bedingung, dass mindestens einer der Reste $R_1$, $R_2$, $R_3$ oder $R_4$, sowie mindestens einer der Reste $R_8$, $R_6$, $R_7$ oder $R_8$ Wasserstoff darstellen, $R_1$ zusätzlich

einen Rest der Formel II, $R_2$ zusätzlich einen Rest der Formel III, $R_3$ zusätzlich einen Rest der Formel IV und $R_4$ zusätzlich einen Rest der Formel V bedeutet, wobei gleichzeitig nur ein Rest der Formel II, III, IV oder V in der Verbindung der Formel I auftritt,

(II) 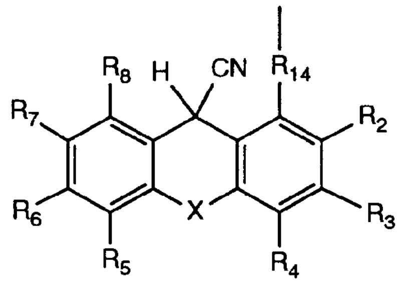 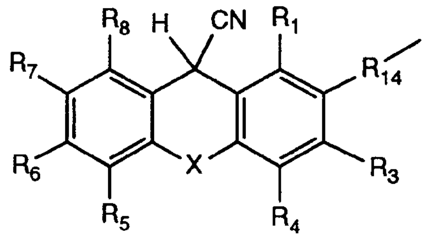 (III)

(IV) 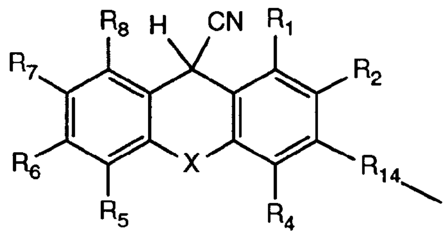 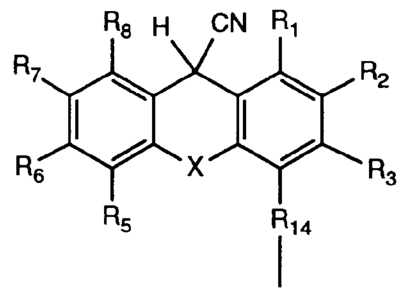 (V)

$R_{11}$ Hydroxy, $C_1$-$C_{12}$-Alkoxy oder

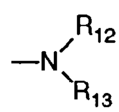

darstellt,
$R_{12}$ und $R_{13}$ unabhängig voneinander Wasserstoff oder $C_1$-$C_{12}$-Alkyl bedeuten,
$R_{14}$ eine direkte Bindung, $C_1$-$C_{12}$-Alkylen, durch Sauerstoff, Schwefel oder $>$N-$R_{10}$ unterbrochenes $C_2$-$C_{12}$-Alkylen;

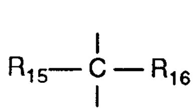 oder 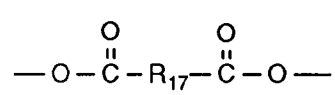

darstellt,
$R_{15}$ und $R_{16}$ unabhängig voneinander Wasserstoff, $C_1$-$C_8$-Alkyl oder Phenyl bedeuten,
oder $R_{15}$ und $R_{16}$ zusammen mit dem C-Atom, an das sie gebunden sind, einen unsubstituierten oder durch 1 bis 3 $C_1$-$C_4$-Alkyl substituierten $C_5$-$C_7$-Cycloalkylidenring bilden,
$R_{17}$ eine direkte Bindung, $C_1$-$C_{12}$-Alkylen, durch Sauerstoff, Schwefel oder $>$N-$R_{10}$ unterbrochenes $C_2$-$C_{12}$-Alkylen; $C_2$-$C_{12}$-Alkenylen, $C_2$-$C_{16}$-Alkyliden, $C_7$-$C_{12}$-Phenylalkyliden, $C_5$-$C_7$-Cycloalkylen, unsubstituiertes oder durch $C_1$-$C_4$-Alkyl substituiertes Phenylen, oder

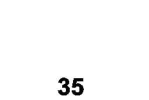

darstellt,
$R_{18}$ Wasserstoff, $C_1$-$C_{18}$-Alkyl, durch Sauerstoff, Schwefel oder $>$N-$R_{10}$ unterbrochenes $C_2$-$C_{18}$-Alkyl; unsubstituiertes oder durch $C_1$-$C_4$-Alkyl substituiertes $C_5$-$C_7$-Cycloalkyl; unsubstituiertes oder durch $C_1$-$C_4$-Alkyl substituiertes Phenyl; $C_7$-$C_9$-Phenylalkyl, Hydroxy, $C_1$-$C_{12}$-Alkoxy, durch Sauerstoff, Schwefel oder $>$N-$R_{10}$ unterbrochenes $C_3$-$C_{12}$-Alkoxy; $C_1$-$C_{18}$-Alkanoyl, durch Sauerstoff, Schwefel oder $>$N-$R_{10}$ unterbrochenes $C_3$-$C_{18}$-Alkanoyl; $C_3$-$C_{18}$-Alkenoyl, Benzoyl oder durch $C_1$-$C_8$-Alkyl substituiertes Benzoyl; Cyano, $-(CH_2)_mCOR_{11}$ oder

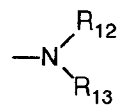

bedeutet, und
$R_{19}$ $C_1$-$C_{18}$-Alkyl, Benzyl oder $-CH_2CH_2OH$ darstellt.

3. Zusammensetzung gemäss Anspruch 1, worin $R_5$, $R_5$, $R_7$ und $R_8$ Wasserstoff bedeuten.

4. Zusammensetzung gemäss Anspruch 1, worin X eine direkte Bindung, Sauerstoff, Schwefel, $-CH_2CH_2-$, -CH=CH-, $>$N-$R_{18}$ oder

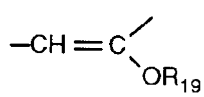

bedeutet,
$R_{18}$ $C_1$-$C_4$-Alkyl ist, und
$R_{19}$ $C_1$-$C_8$-Alkyl oder $-CH_2CH_2OH$ darstellt.

5. Zusammensetzung gemäss Anspruch 1, worin
$R_1$, $R_2$, $R_3$ und $R_4$ unabhängig voneinander Wasserstoff, Chlor, $C_1$-$C_{18}$-Alkyl, durch Sauerstoff unterbrochenes $C_2$-$C_{18}$-Alkyl; $C_5$-$C_8$-Cycloalkyl, Phenyl, $C_7$-$C_9$-Phenylalkyl, Hydroxy, $C_1$-$C_{18}$-Alkoxy, durch Sauerstoff unterbrochenes $C_3$-$C_{18}$-Alkoxy; $C_1$-$C_{18}$-Alkylthio, $C_1$-$C_{18}$-Alkanoyloxy, durch Sauerstoff unterbrochenes $C_3$-$C_{18}$-Alkanoyloxy; $C_3$-$C_{18}$-Alkenoyloxy, Benzoyloxy, Cyano, $-(CH_2)_mCOR_{11}$ oder

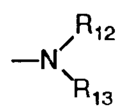

bedeuten, mit der Bedingung, dass mindestens einer der Reste $R_1$, $R_2$, $R_3$ oder $R_4$ Wasserstoff darstellt, $R_2$ zusätzlich einen Rest der Formel III und $R_4$ zusätzlich einen Rest der Formel V bedeutet, wobei gleichzeitig nur ein Rest der Formel III oder V in der Verbindung der Formel I auftritt,

(III) 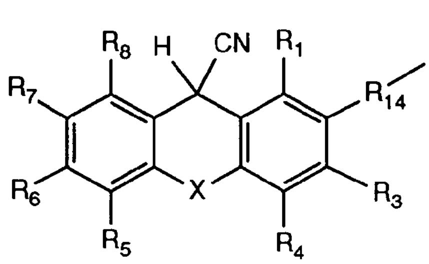

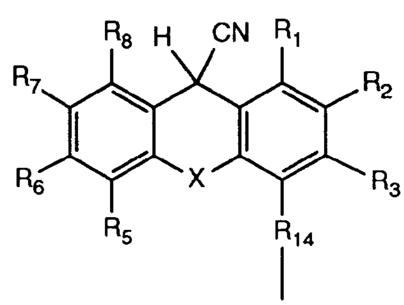 (V)

$R_5$, $R_6$, $R_7$ und $R_5$ unabhängig voneinander Wasserstoff, Chlor, $C_1$-$C_{18}$-Alkyl, $C_5$-$C_8$-Cycloalkyl, Phenyl, Benzyl, Hydroxy, $C_1$-$C_{18}$-Alkoxy, $C_1$-$C_{18}$-Alkylthio, $C_1$-$C_{18}$-Alkanoyloxy, $C_3$-$C_{18}$-Alkenoyloxy, Benzoyloxy,

Cyano, $-(CH_2)_mCOR_{11}$ oder

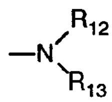

bedeuten, mit der Bedingung, dass mindestens einer der Reste $R_5$, $R_6$, $R_7$ oder $R_5$ Wasserstoff darstellt, $R_{11}$ $C_1$-$C_{12}$-Alkoxy oder

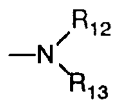

bedeutet,
$R_{12}$ und $R_{13}$ unabhängig voneinander Wasserstoff oder $C_1$-$C_8$-Alkyl darstellen,
$R_{14}$ $C_1$-$C_8$-Alkylen, durch Sauerstoff unterbrochenes $C_2$-$C_{12}$-Alkylen;

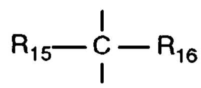

oder

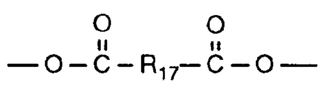

bedeutet,
$R_{15}$ und $R_{16}$ unabhängig voneinander Wasserstoff, $C_1$-$C_8$-Alkyl oder Phenyl darstellen,
oder $R_{15}$ und $R_{16}$ zusammen mit dem C-Atom, an das sie gebunden sind, einen $C_5$-$C_7$-Cycloalkylidenring bilden, und
$R_{17}$ $C_1$-$C_8$-Alkylen, durch Sauerstoff unterbrochenes $C_2$-$C_8$-Alkylen; $C_2$-$C_8$-Alkenylen, $C_2$-$C_8$-Alkyliden, $C_5$-$C_7$-Cycloalkylen oder Phenylen bedeutet.

**6.** Zusammensetzung gemäss Anspruch 1, worin
$R_1$, $R_2$, $R_3$ und $R_4$ unabhängig voneinander Wasserstoff, $C_1$-$C_{18}$-Alkyl, $C_5$-$C_8$-Cycloalkyl, Phenyl, $C_7$-$C_9$-Phenylalkyl, Hydroxy, $C_1$-$C_{18}$-Alkoxy, $C_1$-$C_{18}$-Alkanoyloxy, $C_3$-$C_{18}$-Alkenoyloxy, Benzoyloxy, Cyano oder $-(CH_2)_mCOR_{11}$ bedeuten, mit der Bedingung, dass mindestens einer der Reste $R_1$, $R_2$, $R_3$ oder $R_4$ Wasserstoff darstellt, $R_2$ zusätzlich einen Rest der Formel III und $R_4$ zusätzlich einen Rest der Formel V bedeutet, wobei gleichzeitig nur ein Rest der Formel III oder V in der Verbindung der Formel I auftritt,

(III) 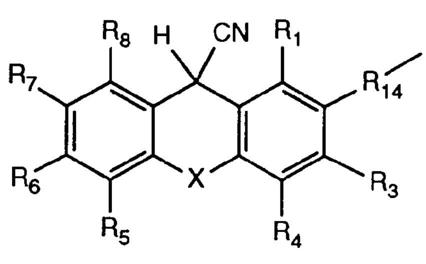 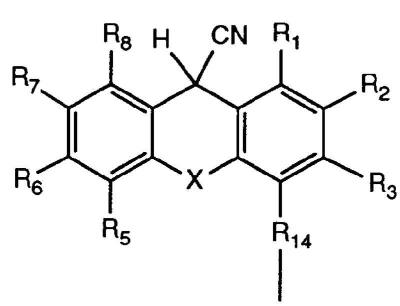 (V)

$R_5$, $R_6$, $R_7$ und $R_5$ unabhängig voneinander Wasserstoff, Chlor, $C_1$-$C_{18}$-Alkyl, $C_5$-$C_8$-Cycloalkyl, Phenyl, Benzyl oder Cyano bedeuten, mit der Bedingung, dass mindestens einer der Reste $R_5$, $R_5$, $R_7$ oder $R_5$ Wasserstoff darstellt,
$R_{11}$ $C_1$-$C_{12}$-Alkoxy ist,

$R_{14}$ $C_1$-$C_8$-Alkylen, durch Sauerstoff unterbrochenes $C_2$-$C_{12}$-Alkylen oder

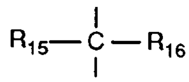

bedeutet,
$R_{15}$ und $R_{16}$ unabhängig voneinander Wasserstoff, $C_1$-$C_8$-Alkyl oder Phenyl darstellen,
oder $R_{15}$ und $R_{16}$ zusammen mit dem C-Atom, an das sie gebunden sind, einen $C_5$-$C_7$-Cycloalkylidenring bilden,
$R_{18}$ Wasserstoff, $C_1$-$C_{12}$-Alkyl, Benzyl, Hydroxy, $C_1$-$C_{12}$-Alkoxy, $C_1$-$C_{18}$-Alkanoyl oder Benzoyl bedeutet,
X eine direkte Bindung, Sauerstoff, Schwefel, $-CH_2CH_2-$, -CH=CH- oder $>N-R_{18}$ darstellt, und
m 0 oder 1 ist.

**7.** Zusammensetzung gemäss Anspruch 1, worin
$R_1$, $R_2$, $R_3$ und $R_4$ unabhängig voneinander Wasserstoff, $C_1$-$C_{12}$-Alkyl, Cyclohexyl, $C_7$-$C_9$-Phenylalkyl, Hydroxy, $C_1$-$C_{12}$-Alkoxy, $C_1$-$C_{12}$-Alkanoyloxy oder $-(CH_2)_mCOR_{11}$ bedeuten, mit der Bedingung, dass mindestens einer der Reste $R_1$, $R_2$, $R_3$ oder $R_4$ Wasserstoff darstellt,
$R_5$, $R_6$, $R_7$ und $R_6$ unabhängig voneinander Wasserstoff, Chlor, $C_1$-$C_8$-Alkyl oder Cyclohexyl bedeuten, mit der Bedingung, dass mindestens einer der Reste $R_5$, $R_6$, $R_7$ oder $R_6$ Wasserstoff darstellt,
$R_{11}$ $C_1$-$C_{12}$-Alkoxy ist,
$R_{18}$ Wasserstoff, $C_1$-$C_8$-Alkyl, Hydroxy, $C_1$-$C_6$-Alkanoyl oder Benzoyl bedeutet,
X eine direkte Bindung, Sauerstoff, Schwefel, $-CH_2CH_2-$, -CH=CH- oder $>N-R_{18}$ darstellt, und
m 0 oder 1 ist.

**8.** Zusammensetzung gemäss Anspruch 1, worin
$R_1$ Wasserstoff, Hydroxy oder $C_1$-$C_{12}$-Alkanoyloxy bedeutet,
$R_2$ Wasserstoff, $C_1$-$C_{12}$-Alkyl, $C_7$-$C_9$-Phenylalkyl, $C_1$-$C_3$-Alkoxy oder $-(CH_2)_mCOR_{11}$ darstellt,
$R_3$ Wasserstoff, Hydroxy oder $C_1$-$C_{12}$-Alkanoyloxy bedeutet,
$R_4$ Wasserstoff, $C_1$-$C_4$-Alkyl oder Cyclohexyl darstellt, mit der Bedingung, dass mindestens einer der Reste $R_1$, $R_2$, $R_3$ oder $R_4$ Wasserstoff ist,
$R_5$, $R_6$, $R_7$ und $R_6$ Wasserstoff bedeuten,
$R_{11}$ $C_1$-$C_{12}$-Alkoxy ist,
$R_{18}$ $C_1$-$C_4$-Alkyl darstellt,
X eine direkte Bindung, Sauerstoff, Schwefel, $-CH_2CH_2-$, -CH=CH- oder $>N-R_{18}$ bedeutet, und
m 0 oder 1 ist.

**9.** Zusammensetzung gemäss Anspruch 1, enthaltend neben den Komponenten (a) und (b) zusätzlich weitere Additive.

**10.** Zusammensetzung gemäss Anspruch 9, enthaltend als weitere Additive phenolische Antioxidantien, Lichtschutzmittel oder/und Verarbeitungsstabilisatoren.

**11.** Zusammensetzung gemäss Anspruch 9, enthaltend als weiteres Additiv mindestens eine Verbindung vom Typ der organischen Phosphite oder Phosphonite.

**12.** Zusammensetzung gemäss Anspruch 1, enthaltend als Komponente a) natürliche, halbsynthetische oder synthetische Polymere.

**13.** Zusammensetzung gemäss Anspruch 1, enthaltend als Komponente a) thermoplastische Polymere, Tackifiers oder Klebstoffe.

**14.** Zusammensetzung gemäss Anspruch 1, enthaltend als Komponente a) ein Polyolefin.

**15.** Zusammensetzung gemäss Anspruch 1, enthaltend als Komponente a) Polyethylen oder Polypropylen.

**16.** Zusammensetzung gemäss Anspruch 1, worin die Komponente b) in einer Menge von 0,0005 bis 5 % bezogen auf das Gewicht der Komponente a) vorliegt.

**17.** Verbindungen der Formel Ia

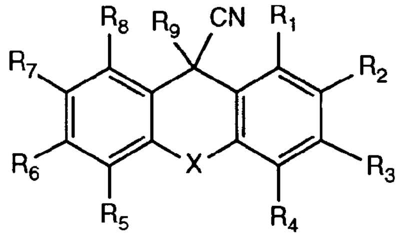

(Ia)

worin $R_1$, $R_2$, $R_3$, $R_4$, $R_5$, $R_6$, $R_7$ und $R_6$ unabhängig voneinander Wasserstoff, $C_1$-$C_{25}$-Alkyl, durch Sauerstoff, Schwefel oder >N-$R_{10}$ unterbrochenes $C_2$-$C_{25}$-Alkyl; unsubstituiertes oder durch $C_1$-$C_4$-Alkyl substituiertes $C_5$-$C_8$-Cycloalkyl; unsubstituiertes oder durch $C_1$-$C_4$-Alkyl substituiertes Phenyl; $C_7$-$C_9$-Phenylalkyl, Hydroxy, $C_1$-$C_{18}$-Alkoxy, durch Sauerstoff, Schwefel oder >N-$R_{10}$ unterbrochenes $C_3$-$C_{18}$-Alkoxy; Mercapto, $C_1$-$C_{18}$-Alkylthio, durch Sauerstoff, Schwefel oder >N-$R_{10}$ unterbrochenes $C_3$-$C_{18}$-Alkylthio; $C_1$-$C_{25}$-Alkanoyloxy, durch Sauerstoff, Schwefel oder >N-$R_{10}$ unterbrochenes $C_3$-$C_{25}$-Alkanoyloxy; $C_3$-$C_{25}$-Alkenoyloxy, Benzoyloxy oder durch $C_1$-$C_{12}$-Alkyl substituiertes Benzoyloxy; Nitro, Cyano, oder $-(CH_2)_mCOR_{11}$ bedeuten, ferner die Reste $R_1$ und $R_2$, $R_2$ und $R_3$, $R_3$ und $R_4$, $R_6$ und $R_6$, $R_6$ und $R_7$ oder $R_7$ und $R_8$ zusammen mit den Kohlenstoffatomen, an die sie gebunden sind, einen Benzoring bilden, oder die gleichen Rest-Paare zusammen $-O(CH_2)_nO-$ bedeuten, mit der Bedingung, dass mindestens einer der Reste $R_1$, $R_2$, $R_3$ oder $R_4$, sowie mindestens einer der Reste $R_5$, $R_6$, $R_7$ oder $R_6$ Wasserstoff darstellen, mindestens einer der Reste $R_1$, $R_2$, $R_3$, $R_4$, $R_5$, $R_6$, $R_7$ und $R_6$ von Wasserstoff verschieden ist, und wenn $R_1$, $R_2$ oder $R_3$ Methyl oder Methoxy bedeutet, mindestens einer der Reste $R_4$, $R_5$, $R_6$, $R_7$ oder $R_8$ von Wasserstoff verschieden ist, $R_1$ zusätzlich einen Rest der Formel IIa, $R_2$ zusätzlich einen Rest der Formel IIIa, $R_3$ zusätzlich einen Rest der Formel IVa und $R_4$ zusätzlich einen Rest der Formel Va bedeutet, wobei gleichzeitig nur ein Rest der Formel IIa, IIIa, IVa oder Va in der Verbindung der Formel Ia auftritt,

(IIa)

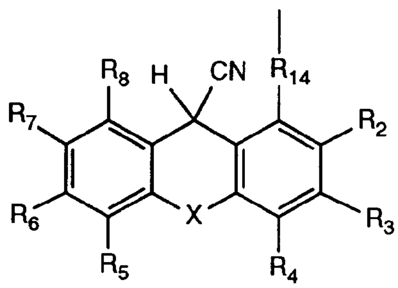

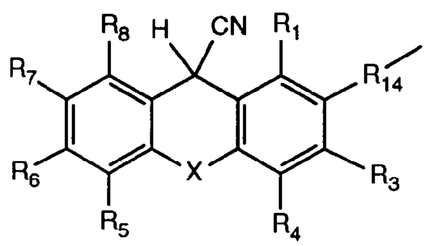

(IIIa)

(IVa)

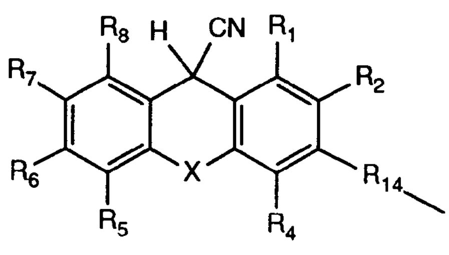

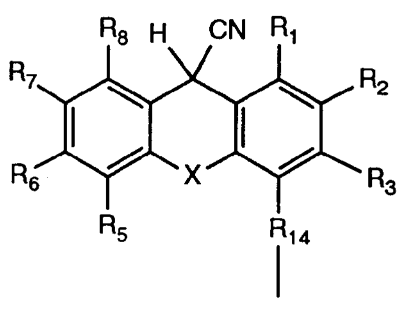

(Va)

$R_9$ Wasserstoff oder einen Rest der Formel VIa

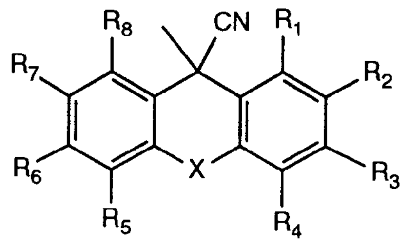

(VIa)

darstellt, wobei $R_1$, $R_2$, $R_3$ und $R_4$ nicht einen Rest der Formel IIa, IIIa, IVa oder Va bedeuten,
$R_{10}$ Wasserstoff oder $C_1$-$C_8$-Alkyl darstellt,
$R_{11}$ Hydroxy, $C_1$-$C_{18}$-Alkoxy oder

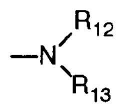

bedeutet,
$R_{12}$ und $R_{13}$ unabhängig voneinander Wasserstoff oder $C_1$-$C_{18}$-Alkyl darstellen,
$R_{14}$ eine direkte Bindung, $C_1$-$C_{18}$-Alkylen, durch Sauerstoff, Schwefel oder $>N-R_{10}$ unterbrochenes $C_2$-$C_{18}$-Alkylen;

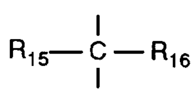 oder 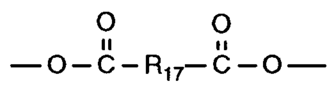

bedeutet,
$R_{15}$ und $R_{16}$ unabhängig voneinander Wasserstoff, $CF_3$, $C_1$-$C_{12}$-Alkyl oder Phenyl darstellen, oder $R_{15}$ und $R_{16}$ zusammen mit dem C-Atom, an das sie gebunden sind, einen unsubstituierten oder durch 1 bis 3 $C_1$-$C_4$-Alkyl substituierten $C_5$-$C_8$-Cycloalkylidenring bilden,
$R_{17}$ eine direkte Bindung, $C_1$-$C_{18}$-Alkylen, durch Sauerstoff, Schwefel oder $>N-R_{10}$ unterbrochenes $C_2$-$C_{18}$-Alkylen; $C_2$-$C_{18}$-Alkenylen, $C_2$-$C_{20}$-Alkyliden, $C_7$-$C_{20}$-Phenylalkyliden, $C_5$-$C_8$-Cycloalkylen, $C_7$-$C_8$-Bicycloalkylen, unsubstituiertes oder durch $C_1$-$C_4$-Alkyl substituiertes Phenylen,

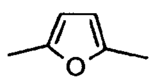 oder 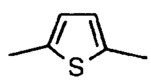

darstellt,
$R_{18}$ Wasserstoff, $C_1$-$C_{25}$-Alkyl, durch Sauerstoff, Schwefel oder $>N-R_{10}$ unterbrochenes $C_2$-$C_{25}$-Alkyl; unsubstituiertes oder durch $C_1$-$C_4$-Alkyl substituiertes $C_5$-$C_8$-Cycloalkyl; unsubstituiertes oder durch $C_1$-$C_4$-Alkyl substituiertes Phenyl; $C_7$-$C_9$-Phenylalkyl, Hydroxy, $C_1$-$C_{18}$-Alkoxy, durch Sauerstoff, Schwefel oder $>N-R_{10}$ unterbrochenes $C_3$-$C_{18}$-Alkoxy; $C_1$-$C_{25}$-Alkanoyl, durch Sauerstoff, Schwefel oder $>N-R_{10}$ unterbrochenes $C_3$-$C_{25}$-Alkanoyl; $C_3$-$C_{25}$-Alkenoyl, Benzoyl oder durch $C_1$-$C_{12}$-Alkyl substituiertes Benzoyl; Cyano, $-(CH_2)_mCOR_{11}$ oder

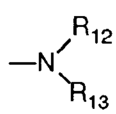

bedeutet,
$R_{19}$ $C_1$-$C_{25}$-Alkyl, $C_7$-$C_9$-Phenylalkyl oder $-CH_2CH_2OH$ darstellt,
X eine direkte Bindung, Sauerstoff, Schwefel, -SO-, $-SO_2-$, $-CH_2-$, $-CH_2CH_2-$, -CH=CH-,

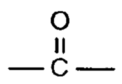 , 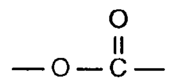 , 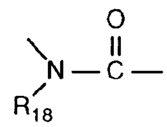 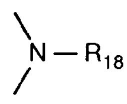 , 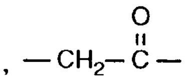

oder

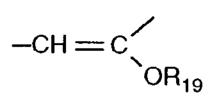

bedeutet,
m 0, 1 oder 2, und
n 1 oder 2 darstellt.

**18.** Verbindungen gemäss Anspruch 17, worin $R_5$, $R_6$, $R_7$ und $R_5$ Wasserstoff bedeuten, mit der Bedingung, dass mindestens einer der Reste $R_1$, $R_2$, $R_3$ oder $R_4$ von Wasserstoff verschieden ist, und keiner der Reste $R_1$, $R_2$ und $R_3$ Methyl oder Methoxy bedeuten.

**19.** Verbindungen gemäss Anspruch 17, worin
$R_1$, $R_2$, $R_3$ und $R_4$ unabhängig voneinander Wasserstoff, $C_4$-$C_{18}$-Alkyl, $C_5$-$C_8$-Cycloalkyl, Phenyl, $C_7$-$C_9$-Phenylalkyl, Hydroxy, $C_3$-$C_{18}$-Alkoxy, $C_1$-$C_{18}$-Alkanoyloxy, $C_3$-$C_{18}$-Alkenoyloxy, Benzoyloxy, Cyano oder $(CH_2)_mCOR_{11}$ bedeuten, mit der Bedingung, dass mindestens einer der Reste $R_1$, $R_2$, $R_3$ oder $R_4$ Wasserstoff darstellt, $R_2$ zusätzlich einen Rest der Formel IIIa und $R_4$ zusätzlich einen Rest der Formel Va bedeutet, wobei gleichzeitig nur ein Rest der Formel IIIa oder Va in der Verbindung der Formel Ia auftritt,

(IIIa) 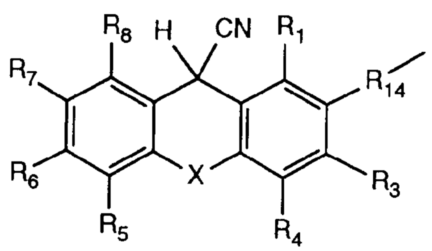 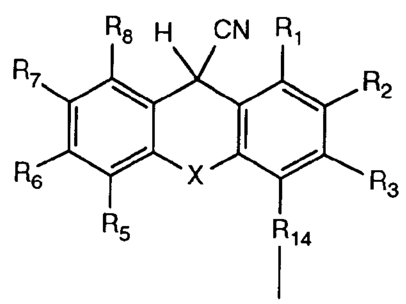 (Va)

$R_5$, $R_5$, $R_7$ und $R_5$ unabhängig voneinander Wasserstoff, $C_1$-$C_{18}$-Alkyl, $C_5$-$C_8$-Cycloalkyl, Phenyl, Benzyl oder Cyano bedeuten, mit der Bedingung, dass mindestens einer der Reste $R_5$, $R_6$, $R_7$ oder $R_8$ Wasserstoff darstellt, und mindestens einer der Reste $R_1$, $R_2$, $R_3$, $R_4$,
$R_5$, $R_6$, $R_7$ und $R_5$ von Wasserstoff verschieden ist,
$R_{11}$ $C_1$-$C_{12}$-Alkoxy ist,
$R_{14}$ $C_1$-$C_8$-Alkylen, durch Sauerstoff unterbrochenes $C_2$-$C_{12}$-Alkylen oder

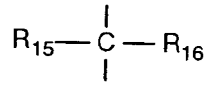

bedeutet,
$R_{15}$ und $R_{16}$ unabhängig voneinander Wasserstoff, $C_1$-$C_8$-Alkyl oder Phenyl darstellen,
oder $R_{15}$ und $R_{16}$ zusammen mit dem C-Atom, an das sie gebunden sind, einen $C_5$-$C_7$-Cycloalkylidenring bilden,
$R_{18}$ Wasserstoff, $C_1$-$C_{12}$-Alkyl, Benzyl, Hydroxy, $C_1$-$C_{12}$-Alkoxy, $C_1$-$C_{18}$-Alkanoyl oder Benzoyl bedeutet,
X eine direkte Bindung, Sauerstoff, Schwefel, $-CH_2CH_2-$, -CH=CH- oder $>N-R_{18}$ darstellt, und
m 0 oder 1 ist.

**20.** Verwendung der Verbindungen der in Anspruch 1 definierten Formel I als Stabilisatoren für organische

Materialien gegen oxidativen, thermischen oder lichtinduzierten Abbau.

**21.** Verfahren zum Stabilisieren eines organischen Materials gegen oxidativen, thermischen oder lichtinduzierten Abbau, dadurch gekennzeichnet, dass man diesem mindestens eine Verbindung der in Anspruch 1 definierten Formel I einverleibt oder auf dieses aufbringt.

Europäisches Patentamt

# EUROPÄISCHER RECHERCHENBERICHT

Nummer der Anmeldung
EP 94 81 0012

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int.Cl.5) |
| A | DATABASE WPI<br>Derwent Publications Ltd., London, GB;<br>AN 84-157885<br>& SU-A-1 047 910 (URALS KIROV POLY) 15. Oktober 1983<br>* Zusammenfassung *<br>--- | 1 | C08K5/00<br>C07C255/47 |
| A | US-A-3 042 674 (JOHN A. FAUST)<br>* Spalte 6 - Spalte 7; Beispiel 8 *<br>----- | 17 | |
| | | | RECHERCHIERTE SACHGEBIETE (Int.Cl.5) |
| | | | C08K<br>C07C |

Der vorliegende Recherchenbericht wurde für alle Patentansprüche erstellt

| Recherchenort | Abschlußdatum der Recherche | Prüfer |
|---|---|---|
| DEN HAAG | 26. April 1994 | Siemens, T |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)